# EUROPÄISCHE PATENTSCHRIFT

(11) **EP 0 831 327 B1**
(45) Veröffentlichungstag und Bekanntmachung des Hinweises auf die Patenterteilung: **09.05.2001**
(21) Anmeldenummer: 97116653.3
(22) Anmeldetag: 24.09.1997
(51) Int. Cl.: G01N 31/22, G01N 33/52, C12Q 1/26, C07D 241/38, C07D 401/14

(54) **Redoxaktive Verbindungen und deren Anwendung**
Redox active compounds and their use
Composés redox-actifs et leur utilisation

(30) Priorität: 24.09.1996 DE 19639169
(43) Veröffentlichungstag der Anmeldung: 25.03.1998
(73) Patentinhaber: Roche Diagnostics GmbH, 68305 Mannheim-Waldhof (DE)
(72) Erfinder: Heindl, Dieter, Dr., 82327 Tutzing (DE); Herrmann, Rupert, Dr., 82362 Weilheim (DE); Hönes, Joachim, Dr., 64673 Zwingenberg (DE); Josel, Hans-Peter, Dr., 82362 Weilheim (DE); Junius-Comer, Martina, Dr., 82327 Tutzing (DE); Merdes, Hartmut, Dr., 69123 Heidelberg (DE); Schmidt, Axel, Dr., 80634 München (DE); Selbertinger, Ernst, Dr., 81675 München (DE)
(74) Vertreter: Weiss, Wolfgang, Dipl.-Chem. Dr.

(56) Entgegenhaltungen:
- EP-A- 0 457 721
- DE-A- 2 016 365
- DE-A- 2 640 695
- JP-A- 8 245 596
- SHETTY, P. S. ET AL: "Redox behavior of some vat dyes at the dropping mercury electrode" INDIAN J. CHEM. (1966), 4(8), 340-3 CODEN: IJOCAP, 1966, XP002052080
- CLARK, MALCOLM COLIN: "Chemistry of indanthrone. XIII. Constitution of indanthren B" J. CHEM. SOC. C (1967), (10), 936-42 CODEN: JSOOAX, 1967, XP002052081
- CHEMICAL ABSTRACTS, vol. 93, no. 15, 1980 Columbus, Ohio, US; abstract no. 150211d, SHAPKIN, V.P. ET AL.: "Synthesis of naphtol[2,3-a]phenazine derivatives." Seite 716; Spalte l; XP002052086 & SHAPKIN, V.P. ETAL.: "Synthesis of Naphto[2,3-a]phenazine derivatives." ZH. ORG. KHIM., Bd. 16, Nr. 5, 1980, Seite 1104
- LOPEZ B., ROSA ET AL: "Synthesis of a new phenanthroline derived ligand with acceptor properties" TETRAHEDRON LETT. (1996), 37(31), 5437-5440 CODEN: TELEAY;ISSN: 0040-4039, 1996, XP002052082
- A. STREITWEISER & C.H. HEATHCOCK: "Introduction to organic chemistry" 1976 , MACMILLAN PUBLISHING CO., INC. , NEW YORK XP002052085 * Seite 1149; Abbildungen *
- AROUNAGUIRI, S. ET AL.: "Redox acitvated luminescence and light-induced nuclease activity of a new mixed-ligand ruthenium(II) complex" PROC. INDIAN ACAD, SCI. (CHEM. SCI)., Bd. 109, Nr. 2, April 1997, Seiten 155-158, XP002052083
- S.G. DIKALOV ET AL.,: "Role of quinone-Iron(III) interaction in NADPH-dependen enzymatic generation of hydroxyl radicals" BIOCHEMISTRY, Bd. 31, 1992, Seiten 8947-8953, XP002052084

## Beschreibung

Die Erfindung betrifft die Verwendung von redoxaktiven Verbindungen zur Herstellung von Nachweisreagenzien für ein Verfahren zur Bestimmung eines Analyten sowie Reagenzienkits, die diese redoxaktiven Verbindungen enthalten. Weiterhin werden neue redoxaktive Verbindungen offenbart.

Redoxreaktionen ermöglichen den Nachweis und die Bestimmung von Analyten in verschiedenen Probenmaterialien. Hierbei wirkt ein oxidierendes oder reduzierendes System direkt oder über einen Mediator auf einen Redoxindikator ein. Die Anwesenheit des Analyten führt zu einer Reduktion oder Oxidation des Redoxindikators. Verwendet man geeignete Redoxindikatoren, bei denen durch Reduktion oder Oxidation eine nachweisbare Änderung der Moleküleigenschaften erfolgt, kann ein qualitativer und/oder quantitativer Nachweis des zu bestimmenden Analyten erfolgen.

Die Bestimmung eines Analyten kann beispielsweise durch ein colorimetrisches Nachweisverfahren, bei dem sich die Farbe des Nachweisreagenz durch Oxidation oder Reduktion verändert, oder durch ein elektrochemisches Nachweisverfahren erfolgen. Beispiele für colorimetrische Nachweisreagenzien sind Resazurine, Heteropolysäuren (EP-B-0 431 456), Tetrazoliumverbindungen (EP-B-0 574 769), nitrosoaromatische Verbindungen (EP-A-0 620 283), RIND-Verbindungen, d.h. Verbindungen, die bei Reduktion eine intramolekulare Reaktion eingehen (vgl. z.B. EP-A-0 190 740 und WO86/04681). Beispiele für redoxaktive Verbindungen bei elektrochemischen Nachweisverfahren sind Nitrosoaromaten, Phenaziniumsalze, Kaliumhexacyanoferrate und Benzochinone (vgl. z.B. EP-A-0 441 222 und EP-A-0 505 494).

Die aus dem Stand der Technik bekannten colorimetrischen Nachweisreagenzien weisen jedoch einige Nachteile auf. So zeigen Tetrazoliumsalze eine ausgeprägte Lichtinstabilität. Weiterhin wird durch Reduktion die Salzstruktur zerstört und die resultierenden Formazane sind meist in Wasser unlöslich (H.-J.Guder, Indigo/Tetrazolium Dyes, Kapitel 12.1 in: Non radioactive labeling and detection of biomolecules, C. Kessler, Hrsg., Springer-Verlag Berlin Heidelberg 1992). Bei Resazurinen ist der Farbunterschied zwischen oxidierter und reduzierter Form für manche Anwendungen zu gering. Heteropolysäuren zeigen aufgrund einer geringen Extinktion und einer ungünstigen Absorptionswellenlänge der reduzierten Form für viele Anwendungen eine nicht ausreichende Sensitivität und sind darüberhinaus hinsichtlich der Absorptionswellenlänge der reduzierten Form wenig variabel. RIND-Verbindungen sind für diagnostische Tests oftmals zu reaktionsträge. Nitrosoaromaten wiederum sind sehr störanfällig und der gebildete Farbstoff kann durch eine Überreduktion verblassen.

Die der vorliegenden Erfindung zugrundeliegende Aufgabe bestand somit darin, neue redoxaktive Verbindungen als Nachweisreagenzien zur Bestimmung von Analyten bereitzustellen, bei denen die Nachteile des Standes der Technik mindestens teilweise beseitigt sind. Die neuen redoxaktiven Verbindungen sollen einerseits hinsichtlich ihrer nachweisbaren Moleküleigenschaften eine hohe Variabilität besitzen und andererseits sowohl in .der reduzierten als auch in der oxidierten Form ausreichend stabil sein.

Die erfindungsgemäße Lösung dieser Aufgabe besteht darin, ein neues in der biochemischen Analytik bzw. medizinischen Diagnostik bisher nicht verwendetes Redoxpaar einzusetzen, das eine Benzochinoxalindion-Substruktur oder Varianten dieser Substruktur enthält, wie z.B. eine Dehydroindanthron- oder Benzonaphthophenazindion-Substruktur. Dehydroindanthrene sind seit langem bekannte Zwischenstufen bei der Synthese von Anthrachinonfarbstoffen wie z.B. halogenierten Indanthronen. Mehrere Vertreter dieser Substanzklasse sind beispielsweise in der Monographienserie "The Chemistry of Heterocyclic Compounds", Band "Phenazines", G.A. Swan und D.G.I. Felton, 1975 Interscience Publishers Inc., New York, insbesondere pp 431 - 433, 469 und 477, beschrieben. Auch die Herstellung von sulfonierten Derivaten ist bekannt (vgl. beispielsweise deutsches Patent Nr. 129847 vom 10. Mai 1901 und deutsches Patent Nr. 216891 vom 13. Dezember 1908).

Verbindungen mit Benzochinoxalin-Substruktur wie z.B. Naphtho [2,3-a] phenazin-8,13-dion, Benzo [a] -naphtho [2,3-h] phenazin-8,13-dion und Varianten mit weiteren ankondensierten Arylresten und deren chemische Reduktion zu Farbstoffen sind ebenfalls seit langem bekannt (siehe "Phenazines", Supra).

Obwohl Redoxpaare mit Benzochinoxalindion-Substruktur nunmehr seit fast 100 Jahren bekannt sind, fanden sie bisher keine Anwendung in der medizinischen Diagnostik bzw. biochemischen Analytik. Überraschenderweise wurde jetzt festgestellt, daß diese Substanzen hervorragend als Nachweisreagenzien insbesondere für colorimetrische Nachweisverfahren geeignet sind. So besitzen sie sowohl im reduzierten als auch im oxidierten Zustand eine hohe Lichtstabilität und können darüberhinaus hinsichtlich der Eigenschaften wie Löslichkeit, Lage der Absorptionsmaxima von oxidierter und reduzierter Form, Redoxpotential usw. durch gezielte Einführung von Substituenten, z.B. hydrophiler Reste, Elektronendonor- oder -akzeptorreste stark variiert werden. Dies ermöglicht ein breites Anwendungsfeld, wie z.B. im Teststreifen oder Flüssigtest. Darüberhinaus ist auch der Einsatz als Mediatoren bei colorimetrischen und elektrochemischen Anwendungen möglich.

Ein Gegenstand der vorliegenden Erfindung ist somit die Verwendung von Verbindungen der Strukturformeln I oder/und II oder Salzen davon als Nachweisreagenzien oder zur Herstellung von Nachweisreagenzien für ein Verfahren zur Bestimmung eines Analyten: 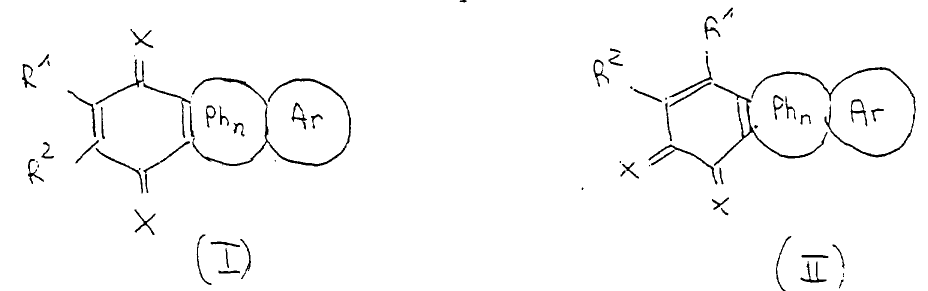 worin R¹ und R² jeweils unabhängig voneinander Wasserstoff, Halogene oder organische Reste sind,
- X: O, S, C(Acc)₂, CH(Acc) oder N(Acc) bedeutet und Acc eine elektronenziehende Gruppe ist,
- Ph: einen gegebenenfalls substituierten Phenylring bedeutet,
- n: eine ganze Zahl von 0 bis 4 ist und
- Ar: eine Gruppe der Strukturformeln IIIa, IIIb, IVa, IVb, Va, Vb, VIa oder VIb bedeutet: 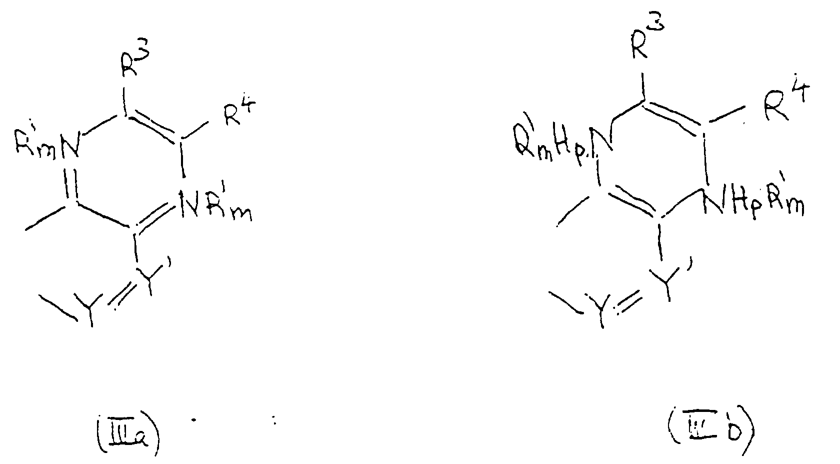 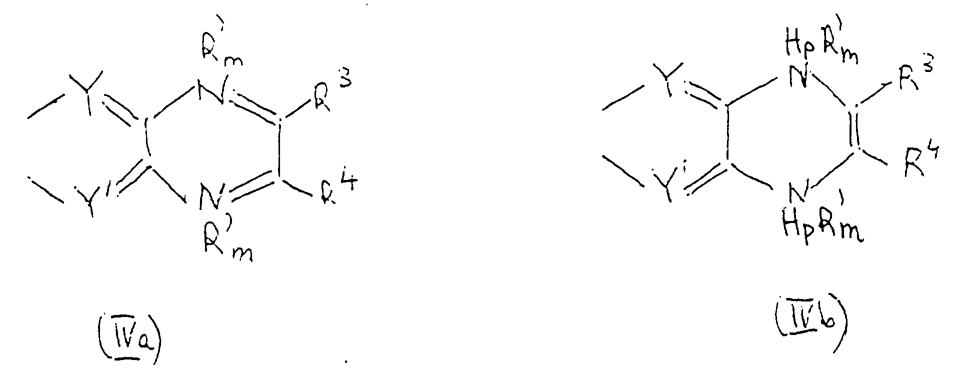 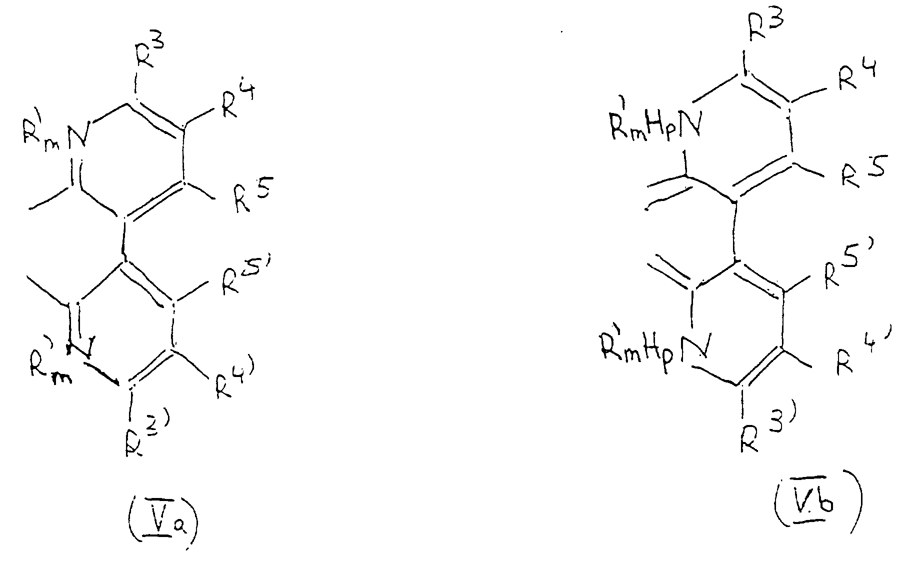 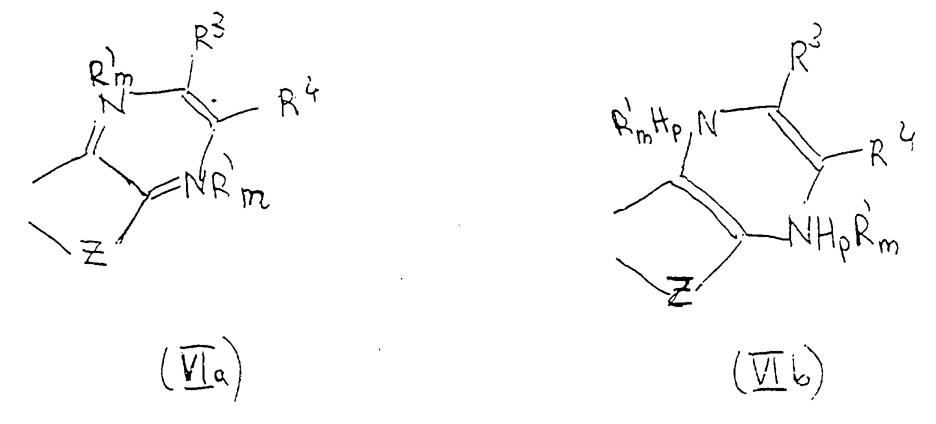
worin R³, R³', R⁴, R⁴', R⁵ und R⁵' jeweils unabhängig voneinander Wasserstoff, Halogen und organische Reste sind,
Y und Y' jeweils unabhängig voneinander N oder CR⁶ sind, wobei
R⁶ Wasserstoff, Halogen oder ein organischer Rest ist,
- Z: NR, O oder S ist, wobei R ein organischer Rest oder Wasserstoff ist,
- R': jeweils unabhängig ein gegebenenfalls substituierter Alkyl- oder Arylrest ist und
- m: jeweils unabhängig 0 oder 1 ist, wobei, wenn m gleich 1 ist, in den Strukturformeln (IIIa), (IVa), (Va) und (VIa) ein an R' gebundener Stickstoff eine positive Ladung trägt und ein entsprechendes Gegenion vorhanden ist, und in den Strukturformeln (IIIb), (IVb), (Vb) und (VIb) p gleich 0 ist und wobei, wenn m gleich 0 ist, p gleich 1 ist.

Neben den Verbindungen der Strukturformeln I und II können auch Salze dieser Verbindungen, z.B. saure Additionssalze, verwendet werden. Beispiele für geeignete Gegenionen für solche Salze oder für geladene Verbindungen mit einem oder mehreren Resten R' sind Halogenide, BF₄⁻, PF₆⁻, ClO₄⁻ oder organische Anionen wie Acetat. Vorzugsweise enthalten geladene Verbindungen nur einen Rest R'.

In den Verbindungen der Strukturformeln I und II umfaßt vorzugsweise zumindest einer der Reste R¹ und R² ein aromatisches oder heteroaromatisches Ringsystem, das gegebenenfalls Substituenten wie etwa z.B. geradkettige, verzweigte, gesättigte oder ungesättigte Kohlenwasserstoffreste, die gegebenenfalls Heteroatome enthalten können, z.B. Alkylreste wie etwa C₁-C₄-Alkyl- oder Haloalkylreste, Hydroxyalkylreste wie etwa C₁-C₄-Hydroxyalkylreste, Halogenatome z.B. F, Cl, Br, I oder/und hydrophile Gruppen - wie im folgenden definiert - tragen kann. Mehrere Substituenten an aromatischen Ringen können auch miteinander, z.B. über Alkylenbrücken, verbrückt sein. R¹ und R² können auch miteinander verbrückt sein. Besonders bevorzugt bilden R¹ und R² zusammen ein aromatisches oder heteroaromatisches Ringsystem, z.B. einen gegebenenfalls substituierten Benzolring. Weiterhin ist möglich, daß R¹ und R² zusammen eine weitere Phₙ-Ar-Gruppe bilden.

Der Rest X in den Formeln I und II ist vorzugsweise O, C(Acc)₂, CH(Acc) oder N(Acc). Acc ist ein elektronenziehender Substituent, d.h. ein Substituent mit einem positiven Hammett-Sigmawert (vgl. z.B. Steric Effects in Organic Chemistry, John Wiley and Sons, Inc. 1956, pp. 570-574 und Progress in Physical Organic Chemistry, Vol. 2, Interscience Publishers, 1964, pp. 333-339). Spezifische Beispiele für solche Substituenten sind Cyano, Carboxy, Nitro, Halogen, Trihalogenmethyl, Carbonyl, Carbamoyl, Sulfonyl, Sulfamoyl, Estergruppen etc. Bevorzugt sind Cyano, Nitro und Estergruppen, insbesondere Cyano und Nitro.

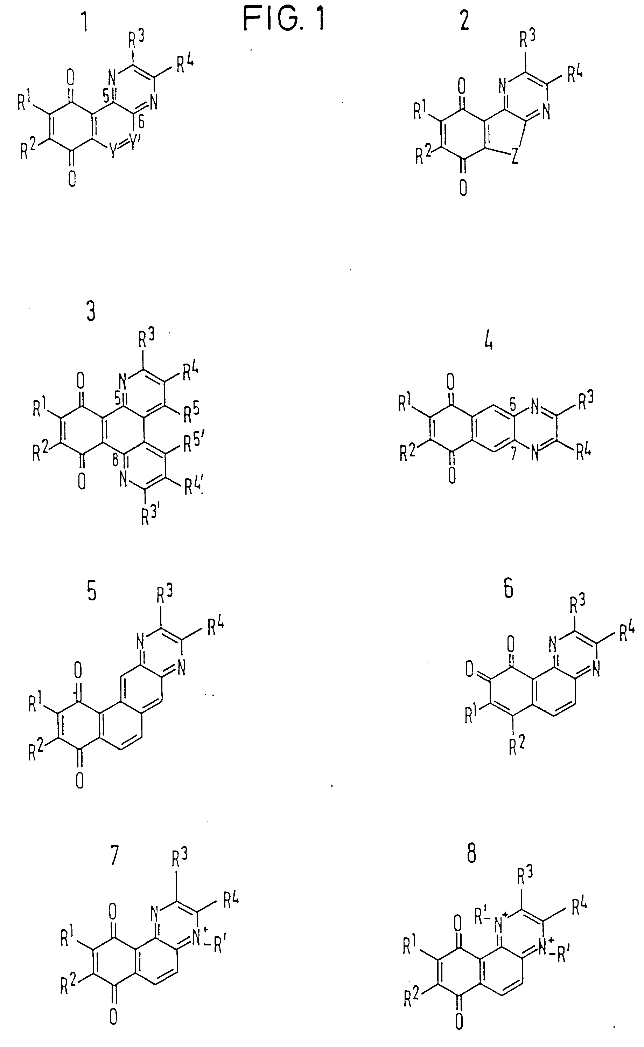

Ph bedeutet einen gegebenenfalls substituierten Phenylring, wobei die Substituenten beispielsweise Halogen, Alkyl oder/und Hydroxyalkyl (siehe oben) sein können. Vorzugsweise ist n eine ganze Zahl von 0 bis 1. Wenn n gleich 0 ist, hat eine Verbindung der Strukturformel I vorzugsweise ein Grundgerüst, wie es in den Verbindungen 1 und 2 in Figur 1 dargestellt ist. Die Verbindung 1 kann dabei als Naphthochinonderivat aufgefaßt werden, bei dem zwei Stickstoffatome als Substituenten an Positionen 5 und 6 des Ringsystems lokalisiert sind. Varianten dieser Strukturen stellen die Verbindungen 3 und 4 in Figur 1 dar. Dort befinden sich die Stickstoffatome an den Positionen 5 und 8 (Verbindung 3) bzw. 6 und 7 (Verbindung 4) des Naphthochinonringsystems.

Eine phenyloge Variante der Verbindung 1 (n = 1) ist Verbindung 5 in Figur 1. Ein Grundgerüst für eine Verbindung der allgemeinen Strukturformel II ist die in Figur 1 dargestellte Verbindung 6.

Die Verbindungen 7 und 8 in Figur 1 sind einfach bzw. zweifach alkylierte Varianten von Verbindung 1. Der Rest R' ist ein gegebenenfalls substituiertes Alkyl, vorzugsweise ein C₁-C₄-und besonders bevorzugt ein C₁-C₂-Alkylrest oder ein gegebenenfalls substituierter Arylrest, vorzugsweise ein Phenylrest.

Die Gruppen der Strukturformeln IIIa und IIIb, IVa und IVb, Va und Vb, VIa und VIb stellen jeweils die oxidierte bzw. reduzierte Variante einer Verbindung dar. In diesen Gruppen umfaßt zumindest einer der Reste R³, R⁴ und - sofern vorhanden - R³', R⁴', R⁵ und R⁵' ein aromatisches oder heteroaromatisches Ringsystem, das gegebenenfalls - entsprechend R¹ und R² - substituiert sein kann. In den Gruppen der Strukturformeln IIIa, IIIb, IVa, IVb, VIa und VIb können R³ und R⁴ miteinander verbrückt sein. Vorzugsweise bilden sie zusammen ein Ringsystem, das zumindest teilweise eine aromatische oder heteroaromatische Struktur aufweist. Ebenfalls ist bevorzugt, wenn einer oder mehrere der Reste, z.B. R³ und R⁴, einzeln ein aromatisches und insbesondere ein heteroaromatisches Ringsystem, z.B. einen Pyridinring enthalten.

In den Gruppen der Strukturformeln Va und Vb können(a) R³ und R⁴ oder/und R³' und R⁴' oder (b) R⁴ und R⁵ oder/und R⁴ und R⁵' jeweils miteinander verbrückt sein. Vorzugsweise bilden sie ein Ringsystem, das zumindest teilweise eine aromatische oder heteroaromatische Struktur aufweist. Besonders bevorzugt bilden die Reste jeweils zusammen ein gegebenenfalls substituiertes Naphthalin- oder Anthrachinonringsystem. Andererseits können auch einer oder mehrere der Reste einzeln ein aromatisches oder heteroaromatisches Ringsystem enthalten.

Ein großer Vorteil der erfindungsgemäßen Verbindungen ist, daß durch eine fast beliebige Substituentenvariation die Eigenschaften einzelner Verbindungen, z.B. die spektrometrischen Eigenschaften wie Lage der Absorptionsmaxima, die elektrochemischen Eigenschaften oder die Löslichkeitseigenschaften, stark variiert werden können. Bevorzugt enthalten die Verbindungen zur Erhöhung der Wasserlöslichkeit eine oder mehrere hydrophile Gruppen, z.B. Ladungsträger, die vorzugsweise ausgewählt sind aus (a) Carbonsäure-, Sulfonsäure- und Phosphonsäuregruppen sowie davon abgeleiteten Salzen, z.B. Ammonium- oder Alkalimetallsalzen, (b) Schwefelsäuremonoester-, Phosphorsäuremonoester- und Phosphorsäurediestergruppen sowie davon abgeleiteten Salzen, (c) und primären, sekundären oder tertiären Amingruppen sowie quaternären Ammoniumgruppen, insbesondere tertiären Amingruppen und quaternären Ammoniumgruppen. Andererseits können die hydrophilen Gruppen auch nichtionische Gruppen sein wie etwa (d) Polyhydroxygruppen, z.B. die Reste eines mehrwertigen Alkohols, eines mehrwertigen Aminoalkohols, z.B. Tris, oder eines Mono-, Oligo- oder Polysaccharids, und (e) C₂-C₃ Polyalkylenoxy- oder C₂-C₃-Polyalkylenthiogruppen. Besonders bevorzugt sind Sulfonsäure- und Amingruppen. Weiterhin können die hydrophilen Gruppen auch eine Kombination von zwei oder mehr der Gruppen (a) - (e) enthalten.

Weiterhin ist es möglich, die Verbindungen der Strukturformel (I) oder/und (II) in Form von Metallkomplexen einzusetzen. Beispiele für geeignete Metalle sind Übergangsmetalle oder Seltenerdmetalle.

Die Herstellung der erfindungsgemäßen Verbindungen kann nach an sich bekannten Methoden erfolgen. So können sulfonierte Dehydroindanthrone durch Sulfonierung von Indanthrenen und anschließende Oxidation erhalten werden. Die Kondensation von Ortho-Chinonen mit Ortho-Diaminoarylverbindungen zu Phenazinen ist eine gängige Reaktion (vgl. G.A.Swan in "The Chemistry of Heterocyclic Compounds: Phenazines, HRSG.A. Weissberger, Interscience Publisher Inc., New York 1975). Die Herstellung von Verbindungen, worin Ar eine Gruppe der Strukturformel (Va) oder (Vb) bedeutet, durch eine Wittig-Reaktion ist beispielsweise in einer Veröffentlichung von Nicolaides und Litinas (J.Chem.Res. (M) 1983, 658-663) beschrieben.

Die erfindungsgemäßen Verbindungen werden zur Herstellung von Nachweisreagenzien für Verfahren zur qualitativen oder/und quantitativen Bestimmung eines Analyten eingesetzt. Diese Bestimmung kann in wäßrigen Flüssigkeiten, z.B. Proben von Körperflüssigkeiten wie etwa Blut, Serum, Plasma oder Urin, Abwasserproben oder Lebensmitteln, durchgeführt werden. Das Verfahren kann sowohl als Naßtest z.B. in einer Küvette oder als Trockentest auf einem entsprechenden Reagenzträger durchgeführt werden.

Die Bestimmung der Analyten kann über eine einzige Reaktion oder durch eine Sequenz von Reaktionen erfolgen. Vorzugsweise umfaßt die Bestimmung des Analyten eine Redoxreaktion, wobei eine nachweisbare Änderung der Moleküleigenschaften der erfindungsgemäßen Nachweisreagenzien, z.B. eine Änderung der spektroskopischen Eigenschaften wie etwa Absorption oder Fluoreszenz, oder eine Änderung der elektrochemischen Eigenschaften erfolgt.

Besonders bevorzugt erfolgt eine Bestimmung der Änderung von Absorptionseigenschaften, die durch spektroskopische Nachweisverfahren in IR-, UV- und insbesondere im sichtbaren Bereich erfolgen kann. Überraschenderweise wurde festgestellt, daß durch Einführung von hydrophilen Gruppen wie etwa Sulfonsäuregruppen die reduzierte Form der erfindungsgemäßen Verbindungen ihr Absorptionsmaximum im langwelligen Bereich von etwa 600 bis 800 nm haben, so daß bei der Bestimmung von Analyten in Serum keine Störungen durch Serumbestandteile auftreten. Dies gilt insbesondere für Gruppen der Strukturformel (III), wobei R³ und R⁴ zusammen ein Naphthalinringsystem bilden und die hydrophilen Gruppen, z.B. Sulfonsäuregruppen, an ein C-Atom gebunden sind, das direkt einem Pyrazin-Stickstoff benachbart ist.

Neben spektrometrischen Eigenschaften können auch elektrochemische Eigenschaften der Nachweisreagenzien bestimmt werden, z.B. Änderungen im Redoxpotential oder im Stromfluß. Beispiele für geeignete elektrochemische Nachweisverfahren sind die Cyclovoltammetrie und die Amperometrie.

Die Analyten, deren Bestimmung durch die erfindungsgemäßen Nachweisreagenzien erfolgt, können beispielsweise Zellen, z.B. Bakterien, Hefen, Pilze, Pflanzenzellen oder Tierzellen wie etwa Leukozyten sein, wobei insbesondere reduzierende oder oxidierende Aktivitäten dieser Zellen nachgewiesen werden können. Weiterhin können Enzyme, z.B. Oxidoreduktasen wie etwa Oxidasen, z.B. Peroxidase, oder Dehydrogenasen, z.B. Glucoseoxidase, Lactat-Dehydrogenase, oder Hydrolasen, z.B. Lipase, Galactosidase, alkalische Phosphatase etc., biologische oder chemische Substanzen, welche mit den Nachweisreagenzien eine Redoxreaktion eingehen können, z.B. Ascorbinsäure, Cystein, Glutation, Thioredoxin etc., metabolisierbare Substanzen, z.B. Glucose, Milchsäure, Triglyceride, Cholesterin etc., immunologisch reaktive Substanzen, z.B. Antigene, Haptene und Antikörper, und Nukleinsäuren oder Nukleinsäurederivate wie peptidische Nukleinsäuren als Analyten nachgewiesen werden.

Demzufolge können die erfindungsgemäßen Verbindungen in zahlreichen Anwendungsgebieten als Nachweisreagenzien eingesetzt werden, z.B. in Flüssig- oder Trockentests für die klinische Diagnostik. Weiterhin können sie in der Histologie zum Nachweis und zur Lokalisierung reduzierender oder oxidierender Enzyme, in der Zellbiologie für Zellproliferations- und Cytotoxizitätstests, ferner für Keimfähigkeitsprüfungen von Saatgut, zur Vitalfärbung, zum histochemischen und cytochemischen Nachweis von Dehydrogenasen in Gewebe, z.B. in Normal- oder Tumorgewebe, und als Nachweismittel in der Dünnschichtchromatographie, z.B. zum Nachweis von Corticosteroiden verwendet werden.

Die Verbindungen der allgemeinen Strukturformeln I und II können als direkte Nachweisreagenzien eingesetzt werden, d.h. die Bestimmung des Analyten erfolgt direkt über eine nachweisbare Änderung der Moleküleigenschaften der Verbindungen. Andererseits können die Verbindungen auch als Mediatoren wirken und gekoppelt mit einem weiteren Nachweissystem eingesetzt werden, d.h. die Bestimmung des Analyten erfolgt nicht direkt über die Änderung der Moleküleigenschaften der Verbindungen, sondern über ein weiteres Nachweissystem, z.B. ein elektrochemisches Nachweissystem (vgl. z.B. EP-A-0 441 222) oder weitere chemische Verbindungen, z.B. Tetrazoliumverbindungen.

In einer bevorzugten Ausführungsform der vorliegenden Erfindung werden die Verbindungen gemäß den Strukturformeln I und II zum Nachweis einer Oxidoreduktase-Aktivität verwendet, wobei der zu bestimmende Analyt einerseits die Oxidoreduktase selbst oder andererseits ein Oxidoreduktase-Substrat sein kann. Der Nachweis erfolgt vorzugsweise in Gegenwart eines Oxidoreduktase-Cosubstrats, insbesondere des Systems NAD (P)⁺ oder NAD(P)H/H⁺, oder aber auch anderer Cosubstrate wie etwa Flavinen oder PQQ.

Die Bestimmung der Oxidoreduktase-Aktivität kann in Gegenwart von Mediatoren erfolgen, welche die Übertragung von Elektronen vom Enzym-Cosubstrat auf die oxidierte Verbindung bzw. die umgekehrte Reaktion, d.h. die Übertragung von Elektronen von der reduzierten Verbindung auf das Enzym-Cosubstrat katalysieren. Beispiele geeigneter Mediatoren für NAD(P) -abhängige Dehydrogenasen sind das Enzym Diaphorase oder N-Methylphenaziniummethosulfat. Es ist jedoch auch eine direkte Übertragung von Elektronen von einem Enzym-Cosubstrat auf die oxidierte Verbindung bzw. die direkte Übertragung von Elektronen von der reduzierten Verbindung auf ein Enzym-Cosubstrat möglich.

Eine weitere bevorzugte Ausführungsform der vorliegenden Erfindung ist die Verwendung von Verbindungen der Strukturformeln I oder/und II zum Nachweis einer Dye(Farbstoff)-Oxidoreduktase-Aktivität, z.B. einer Glucose-Dye-Oxidoreduktase-Aktivität. Hierbei kann die Übertragung von Elektronen direkt von einem Enzymsubstrat auf die oxidierte Verbindung erfolgen. Vorzugsweise wird jedoch ein Coenzym Mediator, z.B. PQQ, verwendet. Zur Bestimmung anderer Analyten kann der Nachweis einer oxidierenden Aktivität auch unter Verwendung der reduzierten Form der erfindungsgemäßen Verbindung erfolgen.

Noch eine weitere bevorzugte Ausführungsform der Erfindung ist die Verwendung von Verbindungen der Strukturformeln I oder/und II zum Nachweis einer Hydrolase-Aktivität, wobei die Hydrolase oder auch ein Hydrolase-Substrat der zu bestimmende Analyt sein kann. Vorzugsweise erfolgt ein solcher Nachweis über ein oxidatives Kupplungssystem, z.B. über eine β-Galactosidasekupplung, wobei die Hydrolyse durch Spaltung eines Hydrolase-Substrats eine oxidierbare Verbindung bildet, bei deren Oxidation die erfindungsgemäße oxidierte Verbindung reduziert wird.

Außerdem betrifft die vorliegende Erfindung ein Verfahren zur Bestimmung eines Analyten in einer Probe, welches dadurch gekennzeichnet ist, daß man die Probe mit einem Reagenz in Kontakt bringt, welches mindestens eine Verbindung der Strukturformeln I oder/und II enthält, und die Bestimmung des Analyten über eine Änderung von nachweisbaren Eigenschaften des Reagenz durchführt. Ein erfindungsgemäßes Reagenz zur Bestimmung eines Analyten enthält mindestens eine Verbindung der Strukturformeln I oder/und II und kann als Bestandteil eines Reagenzienkits vorliegen, der zusätzliche weitere Testkomponenten, z.B. Mediatoren, Begleitsubstanzen wie Puffer, Salze, Detergenzien und gegebenenfalls weitere Hilfsreagenzien in Form von einem Reagenz oder von mehreren separaten Teilreagenzien enthält. Die Begleitsubstanzen können gegebenenfalls so ausgewählt werden, daß sie eine Modifizierung der Moleküleigenschaften, wie etwa der spektralen Charakteristika, der erfindungsgemäßen Verbindungen ermöglichen, z.B. durch pH-Änderung oder/und durch Zusatz von Detergenzien.

Das erfindungsgemäße Reagenz kann ein Reagenz für einen Flüssigtest sein, das z.B. in Form einer Lösung oder Suspension in einer wäßrigen oder nicht wäßrigen Flüssigkeit oder als Pulver oder Lyophilisat vorliegt. Andererseits kann das Reagenz auch als Trockentest-Reagenz z.B. in einem saugfähigen oder quellbaren Träger aufgezogen oder eingebaut in eine lichtempfindliche Filmschicht vorliegen. Ein Flüssigtest-Reagenz enthält vorzugsweise sämtliche für das erfindungsgemäße Verfahren benötigten Komponenten. Als Lösungs- oder Suspendiermittel kommen bevorzugt Wasser, aber auch Mischungen mit wasserlöslichen organischen Lösungsmitteln, wie etwa Alkoholen, Aceton oder Dimethylformamid in Frage. Aus Haltbarkeitsgründen kann es vorteilhaft sein, die für den Test benötigten Komponenten auf zwei oder mehrere Teilreagenzien zu verteilen, die erst bei der eigentlichen Analyse vermischt werden.

Das erfindungsgemäße Reagenz kann auch in Form eines Teststreifens vorliegen. Solche Teststreifen sind in verschiedenen Ausführungsformen bekannt, vgl. z.B. DE-A-32 47 608, EP-A-0 262 445 oder EP-A-0 256 806. In einem Teststreifen liegen die zur Durchführung des Bestimmungsverfahrens benötigten Komponenten des Reagenz auf festen Trägerschichten vor. Als Trägerschichten kommen insbesondere saugfähige oder/und quellbare Materialien in Frage, die von der zu untersuchenden Probeflüssigkeit benetzt werden. Beispiele sind Gelatine, Cellulose, organische Polymere und Copolymere oder Kunstfaservliese. In oder auf diesen Trägermaterialien liegen die Komponenten des Reagenz in fester Form vor. Bei Aufgabe der Probe auf den Teststreifen oder Eintauchen des Teststreifens in die Probe bildet sich in den Streifen ein fiüßiges Milieu aus, innerhalb dem die Nachweisreaktion abläuft. Eine durch die Reaktion verursachte Farbänderung kann visuell oder photometrisch, z.B. reflektometrisch oder auch elektrochemisch ausgewertet werden.

Weiterhin können die erfindungsgemäßen Verbindungen auch in lichtempfindliche Film- bzw. Photoschichten eingebaut werden. In diesem Fall ist die Trägerschicht ein zumindest im wesentlichen transparentes Filmmaterial, das vorzugsweise für elektromagnetische Strahlung der Wellenlängen von 200 - 900 nm durchlässig ist. Geeignete Trägermaterialien sind organische Polymere und Copolymere, z.B. Polystyrole, Polyester, Polycarbonate, Celluloseester etc., bzw. Copolymere davon (vgl. z.B. WO86/04681, Beispiele 11-13).

Die Konzentration der eingesetzten Verbindungen richtet sich nach der Konzentration des zu messenden Analyten. Typische Konzentrationen für die im erfindungsgemäßen Verfahren zu messenden Analyten sind 10⁻⁶ bis 10⁻¹ mol/l im Fall von Substraten oder bis zu 10⁻¹⁵ mol/l beim Nachweis von Enzymaktivitäten. Entsprechend sind typische Konzentrationen der eingesetzten Verbindungen 10⁻⁶ bis 1 mol/l. Sofern weitere nachweisbare Verbindungen, z.B. Tetrazoliumverbindungen oder bestimmte oxidative Kupplungsreagenzien, in Kombination mit den erfindungsgemäßen Verbindungen eingesetzt werden, sind sie vorzugsweise mindestens im stöchiometrischen Verhältnis zu den eingesetzten erfindungsgemäßen Verbindungen vorhanden, besonders bevorzugt in einem 1,5 bis 2-fachen Überschuß.

Ein weiterer Gegenstand der vorliegenden Erfindung sind neue Verbindungen der allgemeinen Strukturformeln I oder II, die mindestens eine hydrophile Gruppe wie in Anspruch 22 definiert enthalten, ausgenommen Indanthrenmonosulfonsäure, Indanthrendisulfonsäure sowie deren Natrium- und Kaliumsalze.

Besonders bevorzugt sind neue Verbindungen mit den allgemeinen Strukturformeln VIIa, VIIb, VIIIa oder VIIIb: 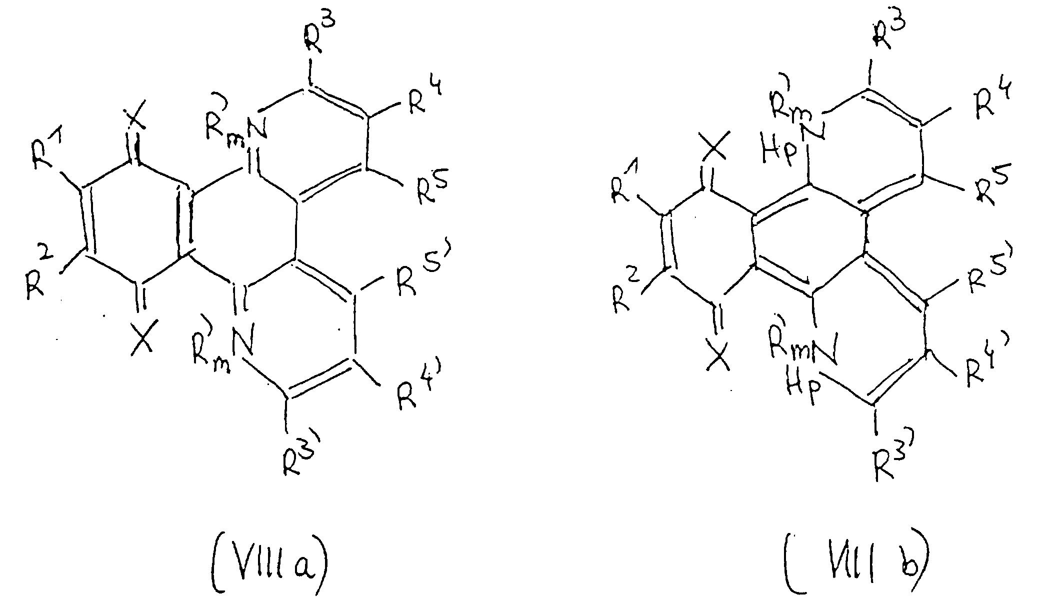 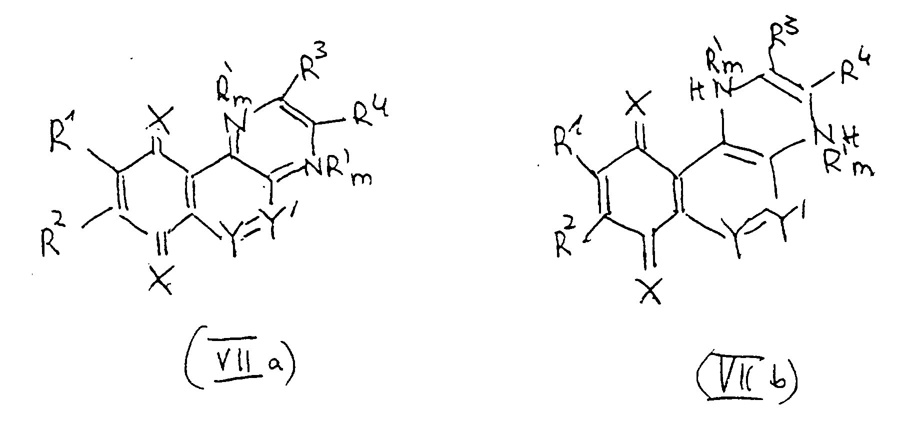 worin R¹, R², X, R', m, p, R³, R⁴, R⁵, R³', R⁴', R⁵', Y und Y' wie in den Verbindungen (I) definiert sind. Besonders bevorzugt bedeutet X gleich O. Weiterhin ist bevorzugt, daß mindestens einer der Reste R¹ und R² oder/und R³, R⁴, R⁵, R³', R⁴', R⁵' ein aromatisches oder heteroaromatisches Ringssystem umfaßt. R¹ und R² können beispielsweise einen gegebenenfalls substituierten Benzolring bilden und in den Verbindungen (VIIa) und (VIIb) können R³ und R⁴ miteinander verbrückt sein und beispielsweise ein gegebenenfalls substituiertes Naphthalin- oder Anthrachinonringsystem bilden. Entsprechend können in den Verbindungen (VIIIa) und (VIIIb) (a) R³ und R⁴ oder/und R³' und R⁴' oder (b) R⁴ und R⁵ oder/und R⁴' und R⁵' miteinander verbrückt sein und beispielsweise ein Ringsystem bilden, das eine aromatische oder heteroaromatische Struktur enthält.

Y und Y' bedeuten vorzugsweise jeweils CR⁶, wobei R⁶ die für die Verbindung (I) angegebene Bedeutung besitzt.

Die Erfindung soll durch die nachfolgenden Abbildungen und Beispiele näher erläutert werden.

Es zeigen:
- Fig. 1: allgemeine Strukturformeln für bevorzugte erfindungsgemäße Indikatorsubstanzen,
- Fig. 2: Strukturformeln der in den Beispielen 1-4 hergestellten Benzonaphthophenazin-8, 13-dione,
- Fig. 3: Strukturformeln weiterer erfindungsgemäßer Benzonaphthophenazin-8,13-dione,
- Fig. 4: Strukturformeln der in den Beispielen 5 und 6 hergestellten Dehydroindanthrensulfonsäuren und des in Beispiel 7 hergestellten Pyridylnaphtho-chinoxalin-7,12-dionmethylsulfats,
- Fig. 5: UV/Vis-Spektren eines erfindungsgemäßen Indikators beim Nachweis von Glucose,
- Fig. 6: UV/Vis-Spektren eines erfindungsgemäßen Indikators beim Nachweis von NADH in Gegenwart des Mediators Diaphorase,
- Fig. 7: ein UV/Vis-Spektrum der Bestimmung von NADH mit einem erfindungsgemäßen Indikator in Gegenwart des Mediators Diaphorase,
- Fig. 8: ein UV/Vis-Spektrum der Kinetik der Reaktion eines erfindungsgemäßen Indikators mit NADH in Gegenwart des Mediators Phenazinmethosulfat,
- Fig. 9: ein UV/Vis-Spektrum eines erfindungsgemäßen Indikators bei einer Bestimmung der β-Galactosidase-Aktivität und
- Fig. 10: einen Vergleich der Lichtstabilität zwischen einem Tetrazoliumsalz und einem erfindungsgemäßen Indikator.

### BEISPIELE

### Beispiele 1 - 4:

### Herstellung von Benzonaphthophenazin-8,13-dionen

Die Herstellung der Verbindungen gemäß den Beispielen 1-4 erfolgt durch Kondensation von Diaminoanthrachinonen mit Ortho-Dicarbonylverbindungen.

### Beispiel 1: 8,13-Dihydro-benzo[a]naphtho[2,3-h] phenazin-8,13-dion-3,6-disulfonsäure-Dinatriumsalz und 10,15-Dihydro-benzo[a] naphtho [2,3 -j] phenazin-10,15-dion-3,6-disulfonsäure-Dinatriumsalz

Eine Suspension von 4.8 g (0.02 mol) 1,2-Diaminoanthrachinon und 7.2 g (0.02 mol) 1,2-Naphthochinon-3,6-disulfonsäure-dinatriumsalz (hergestellt nach W. Langenbeck, H. Le Blanc, B. Lukowcyk. Chem. Ber. 1954, 87, 496; C. Grundmann in Methoden der Organischen Chemie (Houben Weyl) Hrsg. C. Grundmann Georg Thieme Verlag Stuttgart 1979, Band VII 3b (Chinone II) S. 64 - 75) in 100 ml Essigsäure wird 2 h unter Rühren unter Rückfluß zum Sieden erhitzt. Nach dem Abkühlen auf Raumtemperatur wird mit 1000 ml Wasser versetzt. Die Mischung wird filtriert und anschließend das Filtrat dreimal mit Essigester ausgeschüttelt. Die abgetrennte wäßrige Phase wird auf 50 ml eingeengt. Nach Säulenchromatographie an LH 20 mit Wasser als Eluenten erhält man je 1 g der beiden Isomeren.

(Daten für ein Isomeres) :
neg. LSIMS (Wasser/Gly/m-NBA): m/z 542 [m-Na+H], 519 [m-2Na+H], 520 [m-2Na+2H]
UV/Vis-Absorption (0.1 M Phosphatpuffer pH = 7.0): 420 nm; nach Reduktion mit Ascorbinsäure: 780 , 703, 570 nm
1H-NMR (d6-DMSO) ppm: 7.92 (t, [1 H]), 7.99 (t (1 H]), 8.18 (d, [1 H]), 8.20 (d, [1 H]), 8.32 (d, [1 H]), 8.35 (s, [1 H]), 8.58 (d, [1 H]), 8.76 (s, [1 H]), 8.78 (d, 1 H), 9.35 (d, [1H]),
13C-NMR (d6-DMSO) ppm: 125.53, 125.75, 126.06, 126.65, 126.69, 128.64, 130.98, 131.31, 131.58, 133.65, 133.75, 134.75, 135.51, 136.08, 137.98, 140.68, 141.55, 143.11, 143.44, 150.63, 182.35, 183.02

(Daten für anderes Isomeres):
neg. LSIMS (Wasser/Gly/m-NBA): 520, [m-2Na+2H],
1H-NMR (d6-DMSO) ppm: 7.95 (t, [1 H]), 7.08 (t, [1 H]), 8.17 (d, [1H]), 8.19 (d, [1 H] ) , 8.32 (s, [1 H] ) , 8.36 (d [1 H]), 8.68 (d [1 H]), 8.73 (s, [1 H]), 8.73 (d, [1 H]), 9.40 (d, [1H])

UV/Vis-Absorption (0.1 M Phosphatpuffer pH = = 7.0): 414 nm; nach Reduktion mit Ascorbinsäure: 805, 660, 555 nm

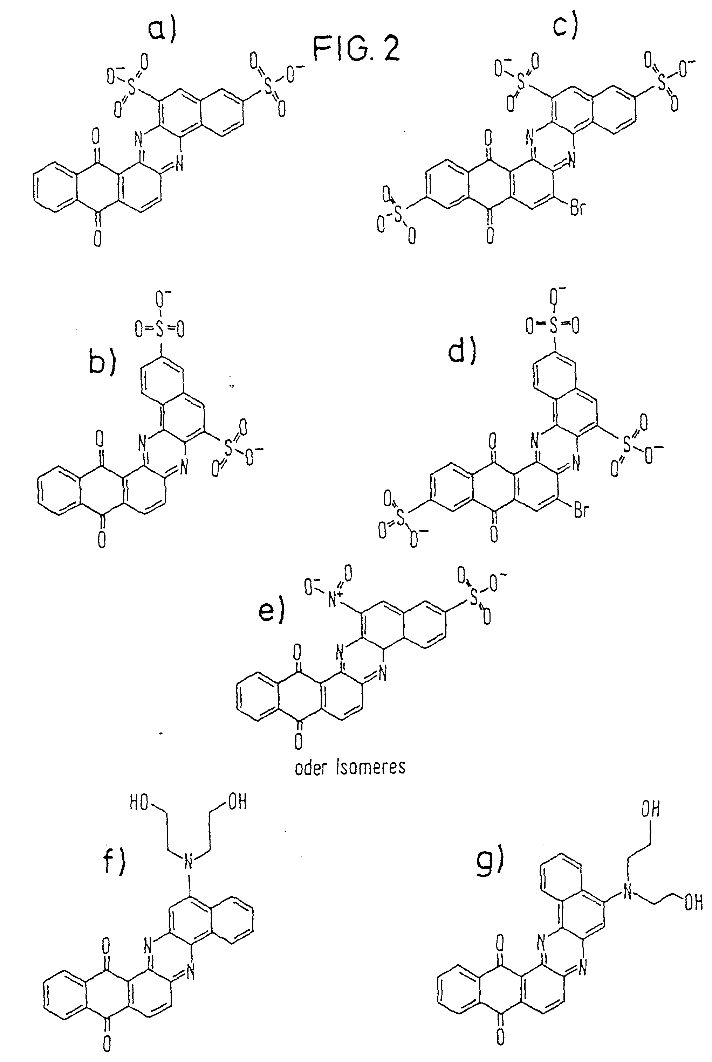

Die Strukturformeln der beiden Isomere sind in Fig. 2a und 2b dargestellt.

### Beispiel 2: 15-Brom-8,13-dihydro-benzo [a] naphtho [2,3-h] phenazin-8,13-dion-3,6,11-trisulfonsäure-trinatriumsalz und 8-Brom-10,15-dihydro-benzo [a] naphtho[2,3-j]phenazin-10,15-dion-3,6,12-trisulfonsäure-trinatriumsalz

Zu einer Lösung von 1 g (3 mmol) 2-Amino-anthrachinon-6-sulfonsäure (O. Bayer in Methoden der Organischen Chemie (Houben Weyl) Hrsg. O. Bayer Georg Thieme Verlag Stuttgart 1979, Band VII 3c (Chinone III) S. 182) in 5 ml 75% Schwefelsäure werden 0.6 ml (15 mmol) Brom gegeben. Es wird 3 h auf 105 °C erwärmt und nach Abkühlen auf Raumtemperatur mit 50 ml Wasser versetzt. 1.5 g des Rohprodukts ( 1,3-Dibrom-2-amino-anthrachinon-6-sulfonsäure) werden ohne Aufreinigung mit 15 ml einer 25 % Ammoniaklösung im Autoklaven bei 100 °C umgesetzt. Das Produkt (1,2-Diamino-3-brom-anthrachinon-6-sulfonsäure) wird durch Zugabe gesättigter Natriumchloridlösung gefällt und durch Säulenchromatographie an LH 20 mit Methanol/Wasser 1 : 1 als Eluenten gereinigt (violette "Bande" wird gesammelt).

Eine Suspension von 400 mg (1 mmol) 1,2-Diamino-3-brom-anthrachinon-6-sulfonsäure und 550 mg (1 mmol) 1,2-Naphthochinon-3,6-disulfonsäure-dinatriumsalz (siehe oben) in 6 ml Essigsäure wird 2 h unter Rühren unter Rückfluß zum Sieden erhitzt. Die Aufarbeitung des Reaktionsgemisches erfolgt analog Bsp. 1. Es werden je 100 mg der beiden Isomeren erhalten.

Daten für ein Isomeres:
neg. LSIMS (Wasser/Gly/m-NBA) : m/z 677 [m-3Na+H] , 678 [m-3Na+2H]
UV/Vis-Absorption (0.1 M Phosphatpuffer pH = = 7.0): 422 nm; nach Reduktion mit Ascorbinsäure: 802 , 716, 660 nm

Daten für anderes Isomeres:
UV/Vis-Absorption (0.1 M Phosphatpuffer pH = = 7.0): 416 nm nach Reduktion mit Ascorbinsäure: 810 (schwach), 715 (schwach), 545 nm

Die Strukturformeln der beiden Isomere sind in Fig. 2c und d dargestellt.

### Beispiel 3: 8,13-Dihydro-3-nitro-benzo[a]naphtho[2,3-h]phenazin-8,l3-dion-6-sulfonsäure oder 10,15-Dihydro-3-nitro-benzo [a] naphtho [2,3-j]phenazin-10,15-dion-3,6-sulfonsäure

6 mmol 5'-Amino-6-hydroxy-naphthalin-2 sulfonsäure (H. E. Fierz-David, L. Blangey, H. Kaul, Helv. Chim. Acta 1946, 29, 1765) werden in 10 ml 68 % Salpetersäure 30 min auf 80 °C erwärmt. Nach schnellem Abkühlen auf 0 °C werden 50 ml gesättigte Natriumchloridlösung zugegeben. Der braungrüne Niederschlag wird abgesaugt und an der Luft getrocknet. Die rohe 3-Nitronaphtho-1,2-chinon-6 sulfonsäure wird mit 3 mmol Diaminoanthrachinon in 20 ml Eisessig 2 h unter Rückfluß zum Sieden erhitzt. Die Aufarbeitung erfolgt wie unter Beispiel 1 beschrieben. Ausbeute: 40 mg (nur ein Isomeres isoliert).
neg. LSIMS (Wasser/Gly/m-NBA): m/z 484 [m-Na],
UV/Vis-Absorption (0.1 M Phosphatpuffer pH = = 7.0): 410 nm; nach Reduktion mit Ascorbinsäure: 800 (schwach), 638 nm

Die Strukturformel der Verbindungen ist in Fig. 2e dargestellt.

### Beispiel 4: 8,13-Dihydro-4-bis(2-hydroxyethyl)amino-benzo[a]naphtho[2,3-h]phenazin-8,13-dion und 10,15-Dihydro-4-bis(2-hydroxyethyl)amino-benzo[a] naphtho [2,3-j]phenazin-10,15-dion

238 mg (1 mol) Diaminoanthrachinon und 261 mg (1 mol) 4-Bis(2-hydroxyethyl)amino-1,2-naphthochinon (hergestellt aus 1,2-Naphthochinon-4-sulfonsäure-Natrium-salz und Diethanolamin nach der Vorschrift in: C. Grundmann in Methoden der Organischen Chemie (Houben Weyl) Hrsg. C. Grundmann Georg Thieme Verlag Stuttgart 1979, Band VII 3b (Chinone II) S. 64 - 75) werden in 20 ml Eisessig 2 h unter Rühren unter Rückfluß zum Sieden erhitzt. Nach Abkühlen auf Raumtemperatur wird die Essigsäure im Vakuum abdestilliert. Der Rückstand wird mit Essigester/Methanol/Eisessigmischung (2 : 2 : 1) als Eluent an Kieselgel getrennt. Die zitronengelbe Bande wird gesammelt.
Ausbeute: 20 mg (Isomerengemisch)
UV/Vis-Absorption (H₂O): 445 nm; nach Reduktion: 548 nm

Die Strukturformeln der beiden Isomere sind in Fig. 2f und 2g dargestellt-.

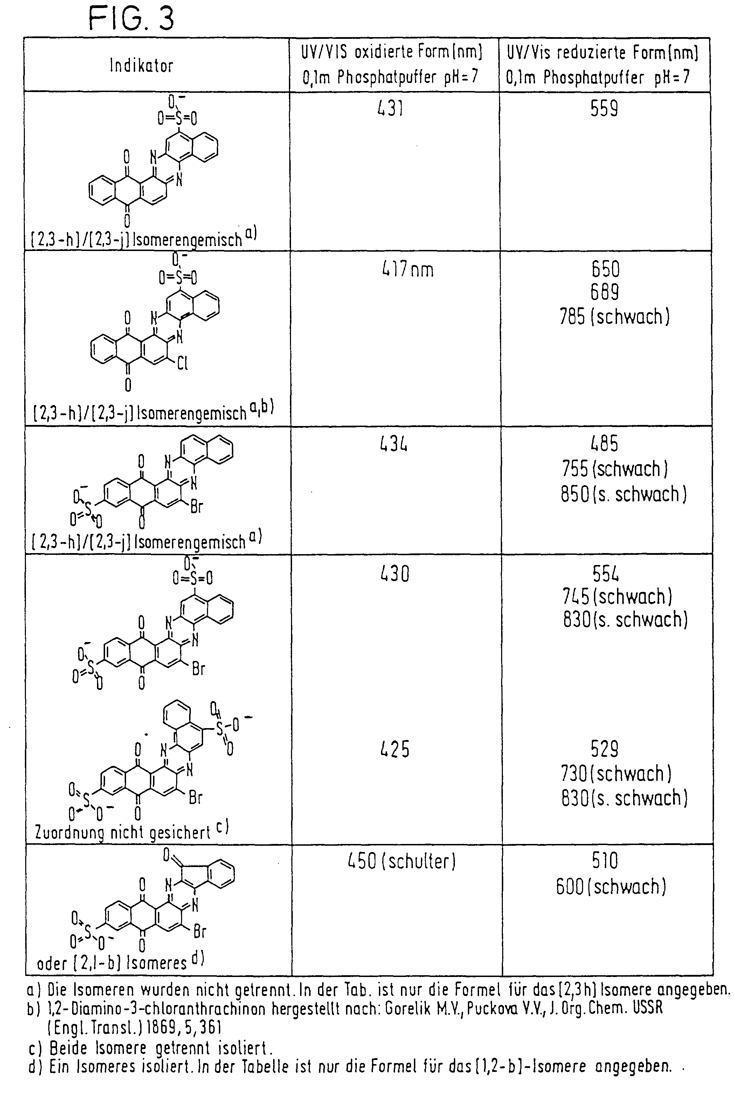

Weitere Verbindungen, die nach den oben beschriebenen Verfahren durch Kondensation von literaturbekannten oder unter Bsp. 1- 4 beschriebenen Diaminoanthrachinonen mit literaturbekannten bzw. kommerziell erhältlichen 1,2-Dicarbonylverbindungen bzw. deren Hydraten erhalten wurden, sind in Fig. 3 zusammengefaßt.

### Beispiele 5-6:

### Herstellung von Dehydroindanthrensulfonsäuren

Dehydroindanthronsulfonsäuren erhält man durch Sulfonierung von Indanthrenen und anschließende Oxidation. Die Verfahren zur Herstellung sind im Lehrbuch Friedländer beschrieben.

### Beispiel 5: Dehydroindanthronmonosulfonsäure (nach Lit.: Fr. IX, 783)

Es werden 0.5 g (8 mmol) Borsäure in 10 ml konz. Schwefelsäure heiß gelöst. Zu der heißen Lösung werden 0.3 g (0.7 mmol) Indanthron zugegeben und die Mischung vier Stunden unter Rückfluß gekocht. Der Ansatz wird abgekühlt und mit 50 ml dest. Eiswasser verdünnt. Anschließend wird filtriert, dabei wird der größte Teil der Schwefelsäure entfernt. Der Rückstand wird in wenig dest. Wasser aufgekocht, heiß filtriert, ausgewaschen und getrocknet. (Ausbeute: 0.25 g Indanthron-Monosulfonsäure) Er wird dann mit einer Mischung aus 10 ml konz. Schwefelsäure und 10 ml rauchender Salpetersäure eine Viertelstunde unter Eiskühlung gerührt. Anschließend wird unter Eiskühlung mit Wasser auf 50 ml verdünnt und abgesaugt. Der braune Feststoff wird an Sephadex LH-20 mit Methanol als Eluent gereinigt. Die Fraktionen, die sich bei Zugabe von Ascorbinsäure blau färben, werden vereinigt. Das Lösungsmittel wird abdestilliert und der Rückstand in Wasser aufgenommen. Die Lösung wird lyophilisiert. Ausbeute: 90 mg
UV/Vis-Absorption: (0.1 M Phosphatpuffer pH = 7.0): 392 nm; nach Reduktion 717, 667 nm.

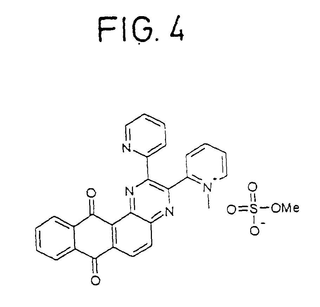

Die Strukturformel der Verbindung ist in Fig. 4 oben (n=1) dargestellt.

### Beispiel 6: Darstellung von Dehydroindanthrondisulfonsäure-Dinatriumsalz ( nach Lit.: Fr. VI, 413)

2 g Indanthron werden mit 1 g Borsäure und 60 ml rauchender Schwefelsäure (Oleum, 30 % SO₃) so lange zum Sieden erhitzt, bis eine Probe der Reaktionslösung mit viel Wasser verdünnt eine klare blaue Lösung ergibt. Anschließend wird die ölige Lösung vorsichtig in 200 ml Eiswasser eingetropft. Die Mischung wird über eine D 4-Glasfritte abgesaugt. Man erhält einen Filterkuchen, der noch Wasser und Schwefelsäure enthält. Die noch feuchte Indanthrondisulfonsäure aus obiger Reaktion wird mit 20 ml rauchender Salpetersäure eine Viertelstunde unter Eiskühlung gerührt. Anschließend wird mit unter Kühlung langsam mit 50 % Natronlauge neutralisiert. Die Lösung wird über Diaion mit Wasser als Eluenten entsalzt. Das braune Eluat wird eingeengt und analog Beispiel 5 an Sephadex LH-20 mit Wasser als Eluent getrennt. Ausbeute: 200 mg
UV/Vis-Absorption: in 0.1 M Phosphatpuffer pH = 7.0): 394 nm; nach Reduktion 790, 714, 655 (sh).

Die Strukturformel der Verbindungen ist in Fig. 4 oben (n=2) dargestellt. Die Substitutionspositionen der Sulfonsäurereste sind vermutlich die Positionen 7 und 16.

### Beispiel 7: 2- (Pyrid-2yl) -3-(1-Methylpyrid-2-ylium)naphtho[2,3-a]chinoxalin-7,12-dionmethylsulfat

2,38 g (10 mmol) 1,2-Diaminoanthrachinon und 2,12 g (10 mmol) Bipyridyl werden in 10 ml Eisessig 5 min unter Rühren unter Rückfluß zum Sieden erhitzt. Nach dem Abkühlen auf Raumtemperatur wird-abgesaugt. Der Rückstand wird aus Eisessig umkristallisiert. (Ausbeute: 2,45 g, 2,3- (Dipyrid-2-yl) -naphtho [2,3.a]chinoxalin-7,12-dione) . Dieser Rückstand (5,84 mmol) wird zusammen mit 0,6 ml (6,42 mmol) Dimethylsulfat in 10 ml Nitrobenzol 30 min auf 100°C erwärmt. Nach Abkühlen auf Raumtemperatur wird durch Zugabe von 50 ml Ether gefällt. Der Rückstand wird abgesaugt und viermal aus Acetonitril mit Ether umgefällt. Er wird in 200 ml siedendem Isopropanol aufgenommen und filtriert. Das Filtrat wird mit Ether/Petrolether 1:1 versetzt. Es fallen feine blaßorange Nädelchen aus. Ausbeute: 250 mg.

Die Strukturformel der Verbindung ist in Figur 4 unten dargestellt.

LSIMS: m/z 429
UV (0,1 M Phosphatpuffer pH=7,0): 380 nm nach Reduktion mit Ascorbinsäure: 533 nm;
1 H-NMR (CD₃CN) ppm: 3,51 (s, [3H], 4,25 (s,[3H]), 7,46 (m, [1 H]), 7,90 (m[3 H]), 8,15 (dd,[2 H]), 8,24 [d, [1 H], 8,29 (dd, [2 H]), 8,55 (dd,[2 H]), 8,74 (d, [1 H] ) , 8,83 (d, [1 H]), 8,94 (d, 1H)
13C-NMR (CD₃CN) ppm: 47,46, 53,65, 124,97, 126,52, 127,27, 127,62, 128,13, 128,65, 128,96, 129,99, 132,88, 134,90, 135,84, 136,11, 138,42, 139,08, 140,70, 143,39, 146,19, 146,67, 147,05, 149,80, 152,02, 153,77, 154,81, 157,83, 183,45, 183,85.

### Beispiel 8: Reaktion eines Indikators mit Glucose/GlucDOR

In einer Küvette werden gemischt:
20 µl einer 5 mM Lösung des Indikators (Dehydroindanthrondisulfonsäure-Dinatriumsalz) in dest. Wasser
1870 µl 0.1 M Phosphatpuffer pH = 7
10 µl 1 M Glucose-Lösung

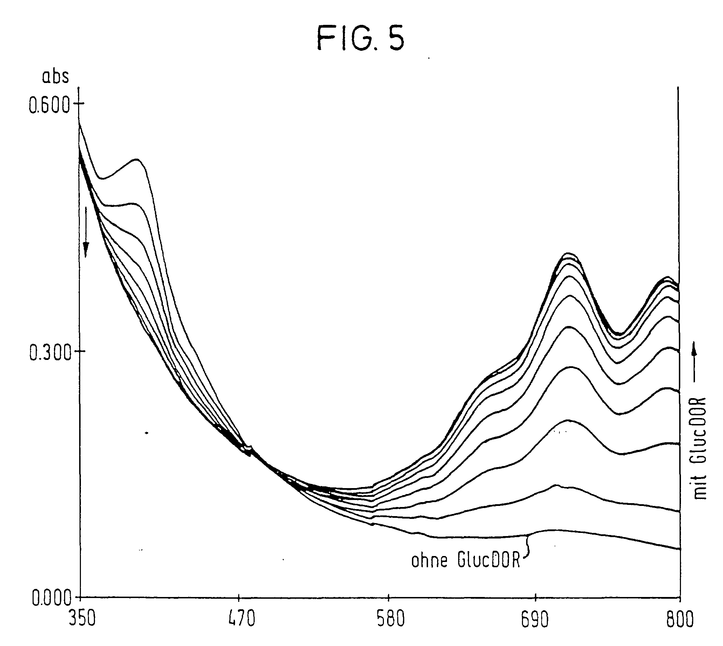

Es werden 100 *µ*l einer GlucDOR Lösung ( in 0.05 M Hepes Puffer pH 7.0 mit PQQ c = 0.016 mg/ml und Calciumchlorid c = = 0.7 mol/l) mit der Konzentration 300 U/ml zugegeben.
Alle 60 sec. nach Zugabe wird ein UV/Vis Spektrum aufgenommen (Fig. 5). Nach 8 min ist keine Farbänderung mehr zu messen.

### Beispiel 9: Reaktion eines Indikators mit NADH/Diaphorase

In einer Küvette werden gemischt:
20 µl einer 5 mM Lösung des 8,13-Dihydro-benzo[a]naphtho[2,3-h]phenazin-8,13-dion-3,6-disulfonsäure-Dinatriumsalzes oder des [2,3-j]-Isomeren in dest. Wasser
1870 µl 0.1 M Phosphatpuffer pH = 7
10 µl einer 0.1 M NADH Lösung in dest. Wasser

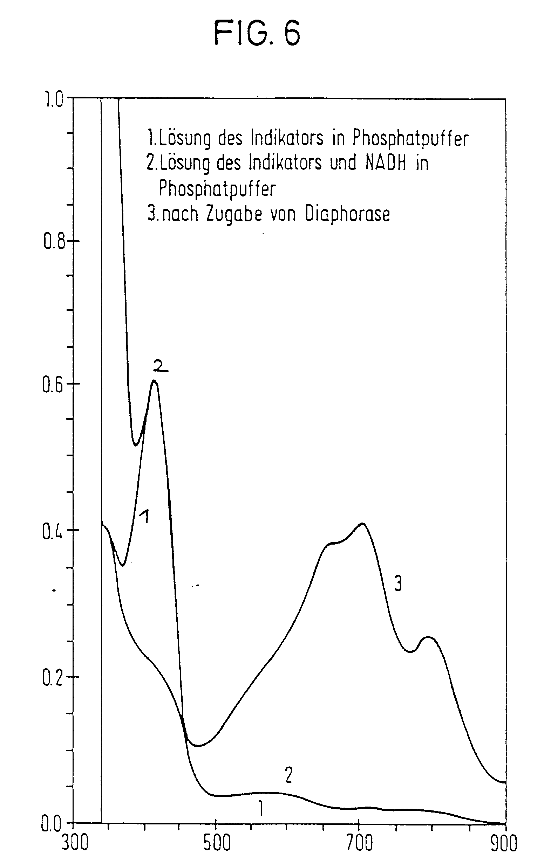

Es werden 100 µl einer Diaphorase-Lösung gelöst in 0.1 M Phosphatpuffer pH = 7 mit der Konz. 100 U/ml zugegeben und nach 60 sec ein UV/Vis Spektrum aufgenommen (Fig. 6).

### Beispiel 10: Bestimmung von NADH

| | |
|---|---|
| Meßansatz | (Endkonzentrationen) |
| | |
| Phosphatpuffer pH = 7 | 100 mM |
| Indikator | 0.5 mM |
| (8,13-Dihydro-benzo[a]naphtho[2,3-h]phenazin-8,13-dion-3,6-disulfonsäure-Dinatriumsalz) oder das [2,3-j] - Isomere | |
| NADH | 50 -100 µmol |

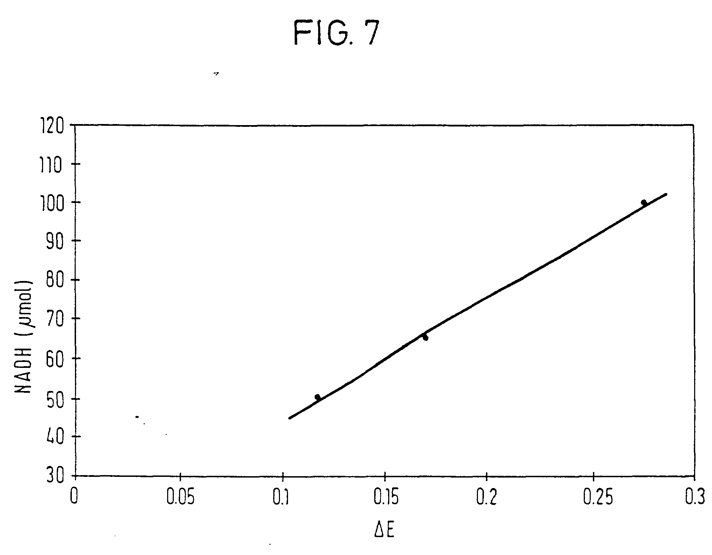

Mit einer Diaphorase Lösung (100 U/ml, 0.1 M Phosphatpuffer pH = 7) wird eine Endkonzentration in der Küvette von 5 U/ml eingestellt und nach einer Minute die Extinktionsänderung bei 704 nm ermittelt. Mit zunehmender NADH-Konzentration wird eine zunehmende Menge eines blaugrünen Farbstoffs gebildet (Fig. 7).

### Beispiel 11: Kinetik der Reaktion eines Indikators mit Phenazinmethosulfat/NADH

In einer Küvette werden nacheinander gemischt:
20 *µ*l einer 5 mM Lösung des 8,13-Dihydro-benzo[a]naphtho [2,3-h]phenazin-8,13-dion-3,6-disulfonsäure-Dinatriumsalzes in dest. Wasser
1870 µl 0.1 M Phosphatpuffer pH = 7
10 µl einer 0.1 M NADH Lösung in dest. Wasser
100 µl einer Phenazinmethosulfat-Lösung gelöst in dest. Wasser mit der Konzentration 10 µg/ml.

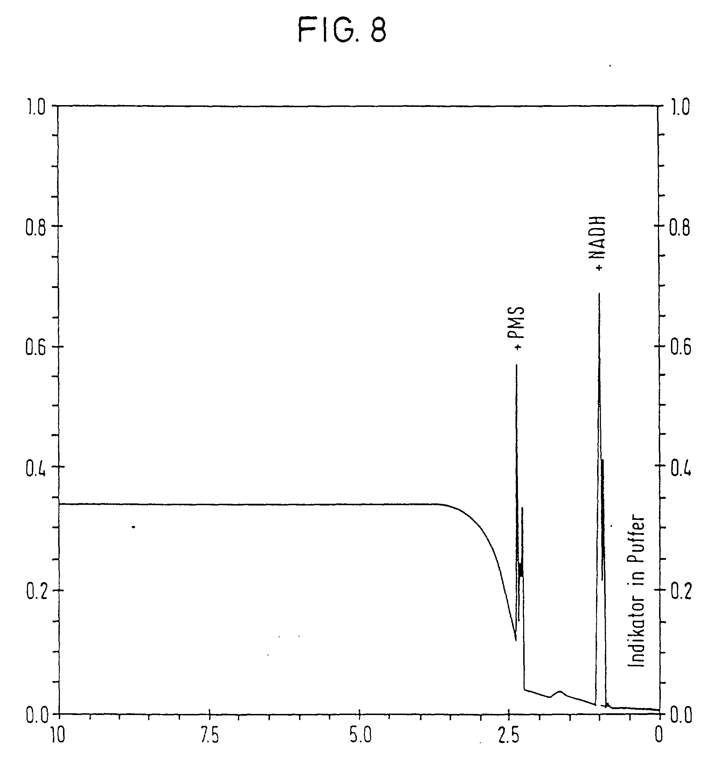

Die Extinktion bei 703 nm wird in Abhängigkeit von der Zeit gemessen (siehe Fig. 8). Nach Zugabe des Mediators wird der blaue Farbstoff gebildet.

### Beispiel 12: Kombination mit einem Hydrolasesubstrat

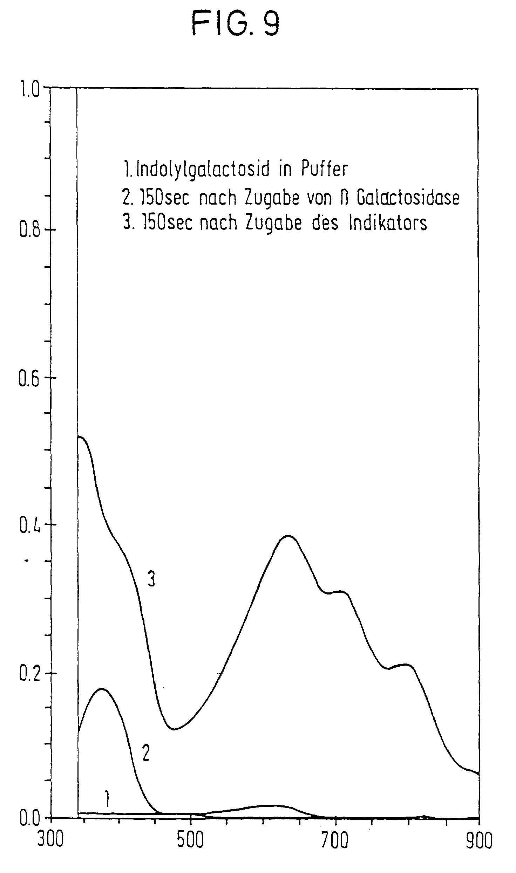

In einer Küvette werden vorgelegt:
20 µl einer 5 mM Lösung von 3-Indolyl-β-D-galactopyranosid
1860 µl 0.1 M Phosphatpuffer pH = 7
Es wird ein UV/Vis Spektrum aufgenommen.
Dann werden 100 *µ*l einer β-Galactosidase Lösung in 0.1 M Phosphatpuffer pH=7 (384 U/ml) zugegeben. 150 sec. nach der Zugabe wird ein UV/Vis Spektrum aufgenommen. Die Absorptionsbande des durch Luftsauerstoff gebildeten Indigos ist schwach zu sehen.
Dann werden 20 µl einer 5 mM Lösung des 8,13-Dihydro-benzo[a]naphtho[2,3-h]phenazin-8,13-dion-3,6-disulfonsäure-Dinatriumsalzes oder des [2,3-j]-Isomeren in dest. Wasser zugegeben. Nach 150 sec wird ein UV/Vis Spektrum aufgenommen. Es bildet sich eine tiefblaue Lösung. Die Absorptionsbanden beider Farbstoffe sind zu erkennen (siehe Fig. 9).

### Beispiel 13: Lichtstabilität von kommerziellem NBT (4-Nitro blue tetrazolium chloride Boehringer Mannheim GmbH 1 087 479 Ch 14528121) und des 8,13-Dihydro-benzo[a]naphtho[2,3-h]phenazin-8,13-dion-3,6-disulfonsäure-Dinatriumsalz oder des [2,3-j]-Isomeren in Standardpuffer des Boehringer Mannheim Testkits (Best Nr. 1101 668) zur Fructosaminbestimmung mit NBT ( J. D. Kruse-Jarres, J. Jarausch et al. Laboratoriumsmedizin 1989, 13, 245)

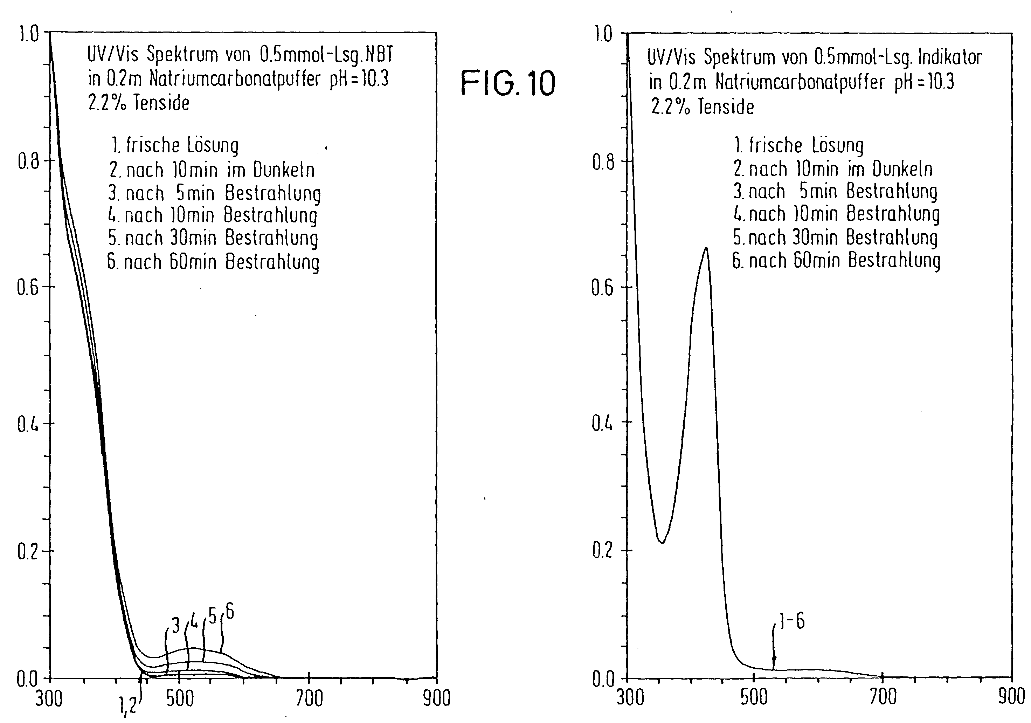

Jeweils 0.5 mM Lösungen von NBT bzw. des Benzonaphthophenazindions in einem 0.2 M Natriumcarbonatpuffer pH = 10.3 mit 2.2 % Tensiden werden frisch hergestellt. Es wird ein UV/Vis Spektrum aufgenommen. Nach 10 min Stehenlassen im Dunkeln bei Raumtemperatur wird ein weiteres Spektrum aufgenommen. Die Spektren verändern sich nicht. Anschließend wird mit einer 60 W Krypton Lampe bestrahlt: Abstand zur Strahlungsquelle 30 cm, Raumtemperatur. Es werden Spektren nach 5, 10, 30, 60 min Gesamtbestrahlungszeit aufgenommen. Im Falle der NBT Lösung verändert sich das UV/Vis Spektrum mit zunehmender Bestrahlungszeit. Mit dem Auge ist eine Violettfärbung beobachtbar. Die Benzonaphthophenazindionlösung verändert sich hingegen nicht (siehe Fig. 10).

## Patentansprüche

1. Verwendung von Verbindungen der Strukturformeln I oder/und II oder Salzen davon zur Herstellung von Nachweisreagenzien für ein Verfahren zur Bestimmung eines Analyten: 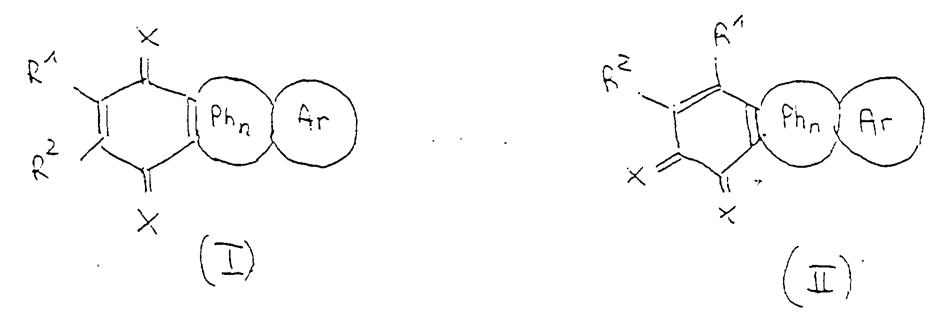 worin R¹ und R² jeweils unabhängig voneinander Wasserstoff, Halogene oder organische Reste sind,
X O, S, C(Acc)₂, CH(Acc) oder N(Acc) bedeutet und Acc eine elektronenziehende Gruppe ist,
Ph einen gegebenenfalls substituierten Phenylring bedeutet,
n eine ganze Zahl von 0 bis 4 ist und
Ar eine Gruppe der Strukturformeln IIIa, IIIb, IVa, IVb, Va, Vb, VIa oder VIb bedeutet: 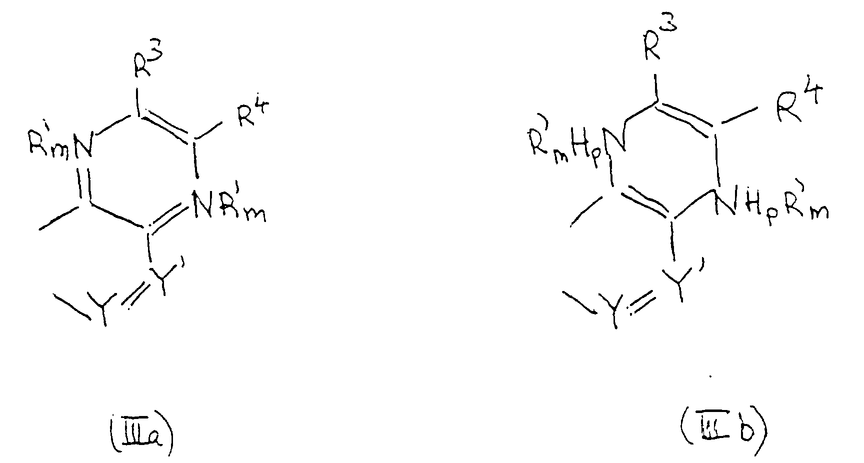 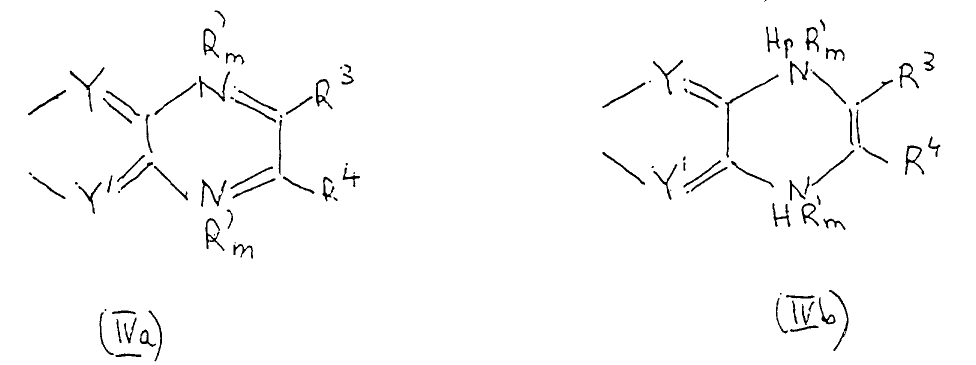 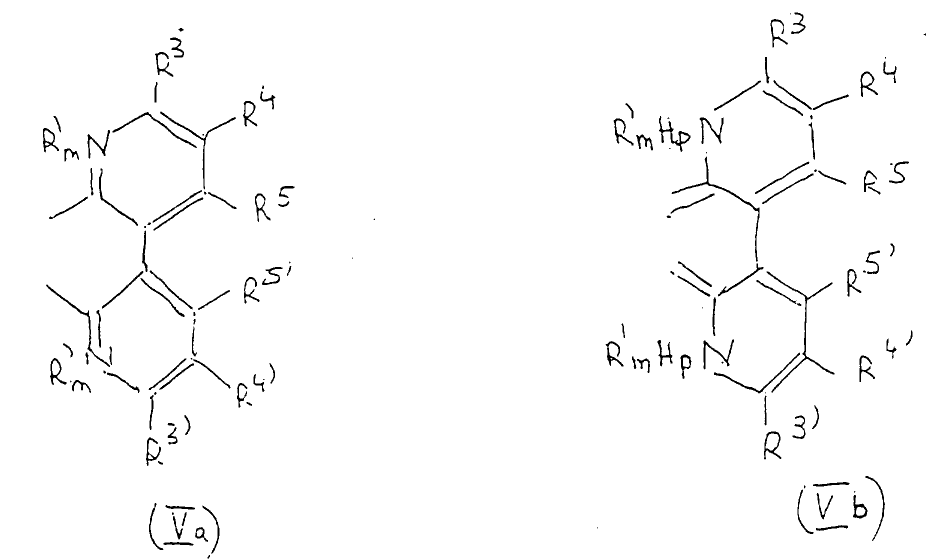 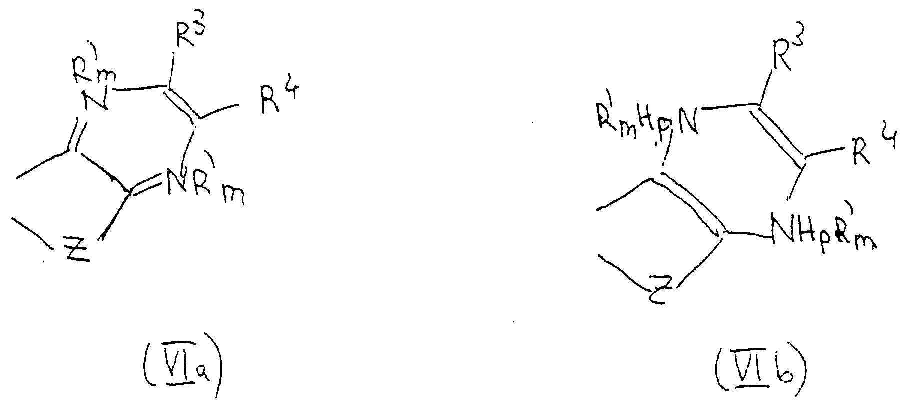
worin R³, R³', R⁴, R⁴', R⁵ und R⁵' jeweils unabhängig voneinander Wasserstoff, Halogen und organische Reste sind,
Y und Y' jeweils unabhängig voneinander N oder CR⁶ sind,
wobei R⁶ Wasserstoff, Halogen oder ein organischer Rest ist,
Z NR, O oder S ist, wobei R ein organischer Rest oder Wasserstoff ist,
R' jeweils unabhängig ein gegebenenfalls substituierter Alkyl- oder Arylrest ist und
m jeweils unabhängig 0 oder 1 ist, wobei, wenn m gleich 1 ist, in den Strukturformeln (IIIa), (IVa), (Va) und (VIa) ein an R' gebundener Stickstoff eine positive Ladung trägt und ein entsprechendes Gegenion vorhanden ist, und in den Strukturformeln (II-Ib), (IVb), (Vb) und (VIb) p gleich 0 ist und wobei, wenn m gleich 0 ist, p gleich 1 ist.

2. Verwendung nach Anspruch 1,
**dadurch gekennzeichnet,**
daß die Bestimmung des Analyten eine Redoxreaktion umfaßt, wobei eine nachweisbare Änderung der Moleküleigenschaften der Nachweisreagenzien erfolgt.

3. Verwendung nach einem der vorgehenden Ansprüche zur Bestimmung einer Oxidoreduktase-Aktivität.

4. Verwendung nach Anspruch 3,
**dadurch gekennzeichnet,**
daß die Bestimmung in Gegenwart eines Oxidoreduktase-Cosubstrats, insbesondere PQQ, NAD(P)⁺ oder NAD(P)H/H⁺, erfolgt.

5. Verwendung nach einem der Ansprüche 3 bis 4,
**dadurch gekennzeichnet,**
daß die Oxidoreduktase der zu bestimmende Analyt ist.

6. Verwendung nach einem der Ansprüche 3 bis 4,
**dadurch gekennzeichnet,**
daß ein Oxidoreduktase-Substrat der zu bestimmende Analyt ist.

7. Verwendung nach einem der Ansprüche 1 bis 2 zur Bestimmung einer Hydrolase-Aktivität.

8. Verwendung nach Anspruch 7,
**dadurch gekennzeichnet,**
daß der Nachweis über ein oxidatives Kupplungssystem erfolgt.

9. Verwendung nach einem der Ansprüche 7 bis 8,
**dadurch gekennzeichnet,**
daß die Hydrolase der zu bestimmende Analyt ist.

10. Verwendung nach einem der Ansprüche 7 bis 8,
**dadurch gekennzeichnet,**
daß ein Hydrolase-Substrat der zu bestimmende Analyt ist.

11. Verwendung nach einem der vorgehenden Ansprüche,
**dadurch gekennzeichnet,**
daß in den Verbindungen der Strukturformeln I und II zumindest einer der Reste R¹ und R² ein aromatisches oder heteroaromatisches Ringsystem umfaßt, das gegebenenfalls substituiert sein kann.

12. Verwendung nach Anspruch 11,
**dadurch gekennzeichnet,**
daß R¹ und R² zusammen ein aromatisches oder heteroaromatisches Ringsystem bilden.

13. Verwendung nach einem der vorgehenden Ansprüche,
**dadurch gekennzeichnet,**
daß in den Gruppen der Strukturformeln IIIa, IIIb, IVa, IVb, Va, Vb, VIa und VIb zumindest einer der Reste R³, R⁴ und - sofern vorhanden - R³', R⁴', R⁵ und R⁵' ein aromatisches oder heteroaromatisches Ringsystem umfaßt, das gegebenenfalls substituiert sein kann.

14. Verwendung nach Anspruch 13,
**dadurch gekennzeichnet,**
daß in den Gruppen der Strukturformeln IIIa, IIIb, IVa, IVb, VIa und VIb R³ und R⁴ miteinander verbrückt sind und vorzugsweise zusammen ein Ringsystem bilden, das zumindest teilweise eine aromatische oder heteroaromatische Struktur aufweist.

15. Verwendung nach Anspruch 13,
**dadurch gekennzeichnet,**
daß in den Gruppen der Strukturformeln Va und Vb (a) R³ und R⁴ oder/und R³' und R⁴' oder (b) R⁴ und R⁵ oder/und R⁴' und R⁵'. jeweils miteinander verbrückt sind und vorzugsweise zusammen ein aromatisches oder heteroaromatisches Ringsystem bilden, das zumindest teilweise eine aromatische oder heteroaromatische Struktur aufweist.

16. Verwendung nach Anspruch 14 oder 15,
**dadurch gekennzeichnet,**
daß die Reste jeweils zusammen ein gegebenenfalls substituiertes Naphthalin- oder Anthrachinonringsystem bilden.

17. Verwendung nach einem der vorgehenden Ansprüche,
**dadurch gekennzeichnet,**
daß man die Verbindungen der Strukturformeln (I) oder/und (II) in Form von Metallkomplexen einsetzt.

18. Verfahren zur Bestimmung eines Analyten in einer Probe,
**dadurch gekennzeichnet,**
daß man die Probe mit einem Reagenz in Kontakt bringt, welches eine Verbindung der Strukturformeln (I) wie in Anspruch 1 definiert oder/und (II) wie in Anspruch 1 definiert enthält, und die Bestimmung des Analyten über eine Änderung von nachweisbaren Eigenschaften des Reagenz durchführt.

19. Reagenz zur Bestimmung eines Analyten in einer Probe,
**dadurch gekennzeichnet,**
daß es mindestens eine Verbindung der Strukturformeln (I) wie in den Ansprüche 22-29 definiert oder/und (II) wie in den Ansprüche 22-29 definiert enthält.

20. Reagenzienkit,
**dadurch gekennzeichnet,**
daß er neben anderen Testkomponenten ein Reagenz nach Anspruch 19 enthält.

21. Reagenzienkit nach Anspruch 20,
**dadurch gekennzeichnet,**
daß er Begleitsubstanzen enthält, die eine Modifizierung der Moleküleigenschaften von Verbindungen der Strukturformel (I) oder/und (II) bewirken.

22. Verbindungen der allgemeinen Strukturformeln (I) oder (II) : 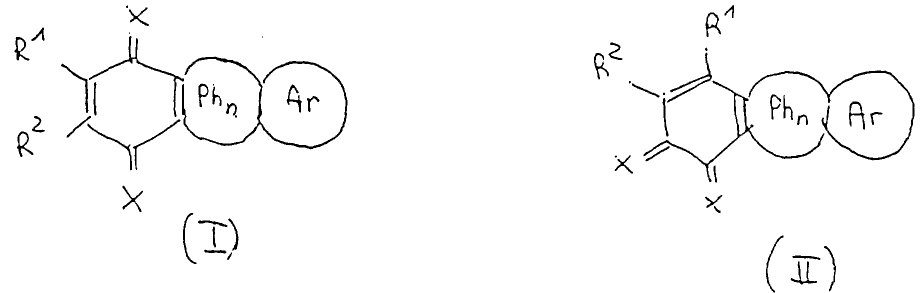 worin R¹, R², X, Ph, n und Ar wie in Anspruch 1 definiert sind und worin die Verbindungen mindestens eine hydrophile Gruppe enthalten, ausgewählt aus (a) Sulfonsäure- und Phosphonsäuregruppen sowie davon abgeleiteten Salzen, (b) Schwefelsäuremonoester-, Phosphorsäurmonoester- und Phosphorsäurediestergruppen sowie davon abgeleiteten Salzen, (c) primären, sekundären und tertiären Amingruppen, quarternären Ammoniumgruppen, (d) Polyhydroxygruppen und (e) C₂-C₃-Polyalkylenoxy- und C₂-C₃-Polyalkylenthiogruppen, ausgenommen Indanthrenmonosulfonsäure, Indanthrendisulfonsäure sowie deren Natrium- und Kaliumsalze.

23. Verbindungen nach Anspruch 22 mit den allgemeinen Strukturformeln (VIIa) oder (VIIb): 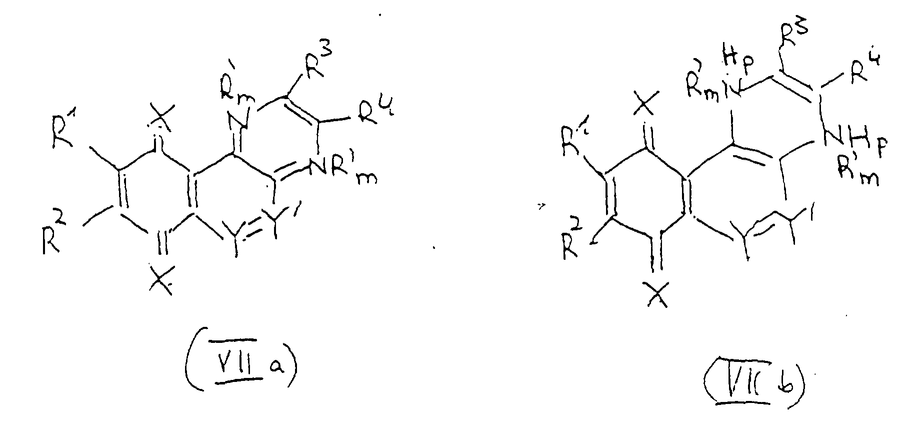 worin R¹, R², X, R', m, p, R³, R⁴, Y und Y' wie in Anspruch 1 definiert sind.

24. Verbindungen nach Anspruch 23 mit den allgemeinen Strukturformeln (VIIIa) oder (VIIIb): 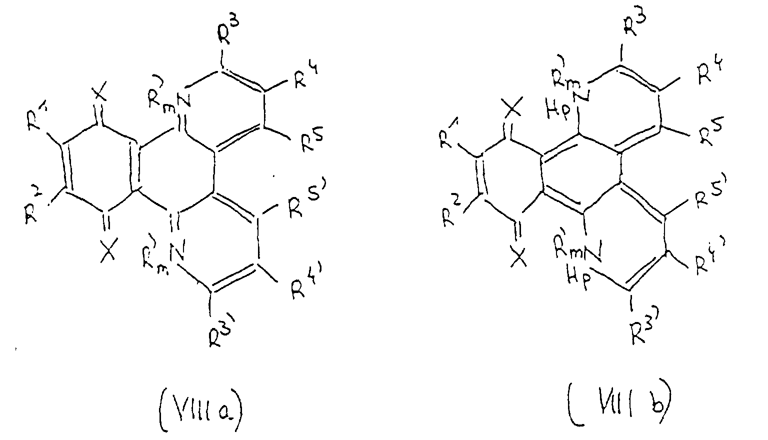 worin R¹, R², X, R', m, p, R³, R⁴, R⁵, R³', R⁴' und R⁵' wie in Anspruch 1 definiert sind.

25. Verbindungen nach einem der Ansprüche 22 - 24,
**dadurch gekennzeichnet,**
daß X gleich O bedeutet.

26. Verbindungen nach einem der Ansprüche 22 - 25,
**dadurch gekennzeichnet,**
daß mindestens einer der Reste R¹ und R² ein aromatisches oder heteroaromatisches Ringsystem umfaßt.

27. Verbindungen nach einem der Ansprüche 23 und 25 - 26,
**dadurch gekennzeichnet,**
daß mindestens einer der Reste R³ und R⁴ ein aromatisches oder heteroaromatisches Ringsystem umfaßt.

28. Verbindungen nach einem der Ansprüche 23 und 25 - 27,
**dadurch gekennzeichnet,**
daß Y und Y' jeweils CR⁶ bedeuten, wobei R⁶ die in Anspruch 1 angegebene Bedeutung besitzt.

29. Verbindungen nach einem der Ansprüche 24 - 28,
**dadurch gekennzeichnet,**
daß mindestens einer der Reste R³, R⁴, R⁵, R³', R⁴', R⁵' ein aromatisches oder heteroaromatisches Ringsystem umfaßt.

## Claims

1. Use of compounds of the structural formulae I and/or II or salts thereof for the production of detection reagents for a method for the determination of an analyte 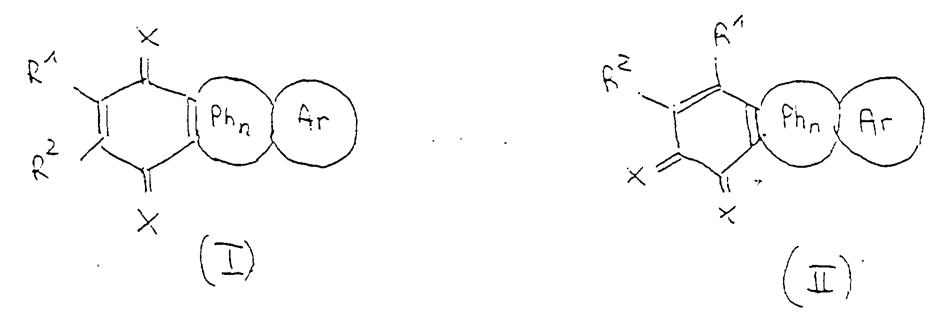 in which R¹ and R² each independently of one another are hydrogen, halogens or organic residues,
X denotes O, S, C (Acc)₂, CH (Acc) or N(Acc) and Acc is an electron-attracting group,
Ph denotes an optionally substituted phenyl ring,
n is an integer from 0 to 4 and
Ar denotes a group of the structural formulae IIIa, IIIb, IVa, IVb, Va, Vb, VIa or VIb: 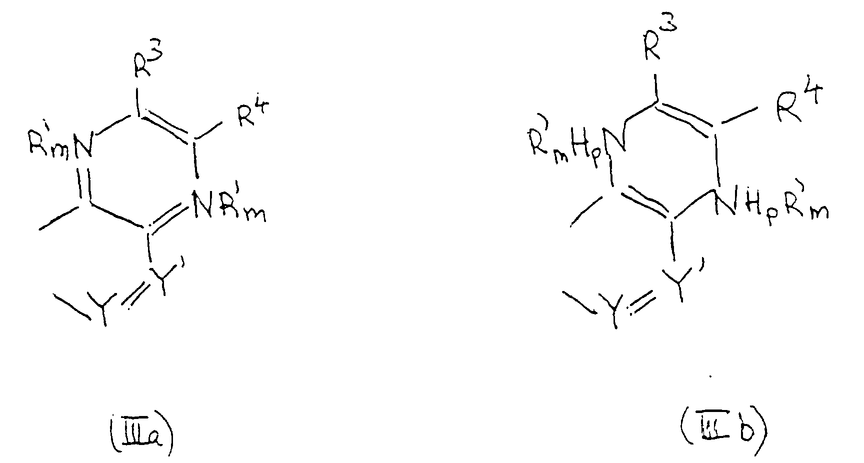 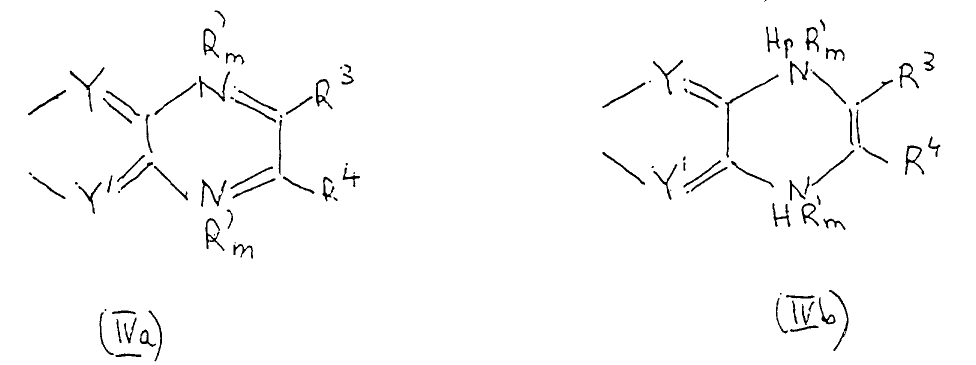 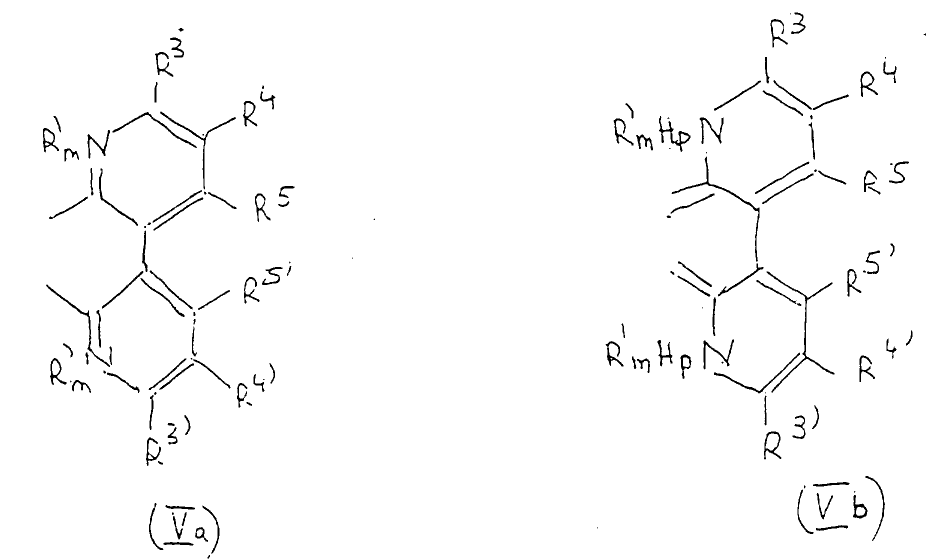 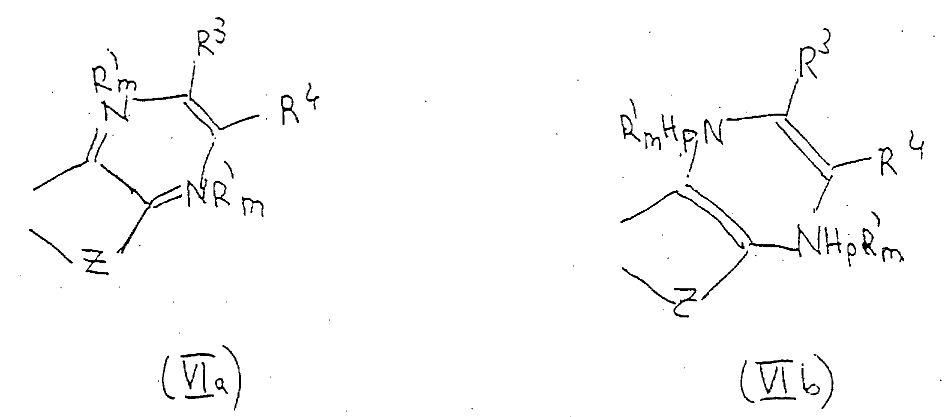
in which R³, R³', R⁴, R⁴', R⁵ and R⁵' each independently of one another are hydrogen, halogen and organic residues,
Y and Y' each independently of one another are N or CR⁶, in which R⁶ is hydrogen, halogen or an organic residue,
Z is NR, O or S, in which R is an organic residue or hydrogen
R' is in each case independently an optionally substituted alkyl or aryl residue and
m is in each case independently 0 or 1 and, if m equals 1, a nitrogen bound to R' in the structural formulae (IIIa), (IVa), (Va) and (VIa) carries a positive charge and an appropriate counterion is present and p equals 0 in the structural formulae (IIIb), (IVb), (Vb) and (VIb) and, if m equals 0, p equals 1.

2. Use as claimed in Claim 1, characterised in that the determination of the analyte comprises a redox reaction in which there is a detectable change of the molecular properties of the detection reagents.

3. Use as claimed in one of the preceding claims for the determination of an oxidoreductase activity.

4. Use as claimed in Claim 3, characterised in that the determination is carried out in the presence of an oxidoreductase cosubstrate, in particular PQQ, NAD(P)⁺ or NAD(P) H/H^{+.}

5. Use as claimed in one of the Claims 3 to 4, characterised in that the oxidoreductase is the analyte to be determined.

6. Use as claimed in one of the Claims 3 to 4, characterised in that an oxidoreductase substrate is the analyte to be determined

7. Use as claimed in one of the Claims 1 to 2 for the determination of a hydrolase activity.

8. Use as claimed in Claim 7, characterised in that the detection is effected via an oxidative coupling system.

9. Use as claimed in one of the Claims 7 to 8, characterised in that the hydrolase is the analyte to be determined.

10. Use as claimed in one of the Claims 7 to 8, characterised in that a hydrolase substrate is the analyte to be determined.

11. Use as claimed in one of the preceding claims, characterised in that at least one of the residues R¹ and R² in the compounds of the structural formulae I and II comprises an aromatic or heteroaromatic ring system which can optionally be substituted.

12. Use as claimed in Claim 11, characterised in that R¹ and R² together form an aromatic or heteroaromatic ring system.

13. Use as claimed in one of the preceding claims, characterised in that in the groups of the structural formulae IIIa, IIIb, IVa, IVb, Va, Vb, VIa and VIb at least one of the residues R³, R⁴ and - if present - R³', R⁴', R⁵ and R⁵' comprise an aromatic or heteroaromatic ring system which can optionally be substituted.

14. Use as claimed in Claim 13, characterised in that in the groups of the structural formulae IIIa, IIIb, IVa, IVb, VIa and VIb R³ and R⁴ are bridged together and preferably form a ring system which at least partially has an aromatic or heteroaromatic structure.

15. Use as claimed in Claim 13, characterised in that in the groups of the structural formulae Va and Vb (a) R³ and R⁴ and/or R³' and R⁴' or (b) R⁴ and R⁵ and/or R⁴' and R⁵' are in each case bridged together and preferably together form an aromatic or heteroaromatic ring system which at least partially has an aromatic or heteroaromatic structure.

16. Use as claimed in Claim 14 or 15, characterised in that the residues in each case together form an optionally substituted naphthalene or anthraquinone ring system.

17. Use as claimed in one of the preceding claims, characterised in that the compounds of the structural formulae (I) and (II) are used in form of metal complexes.

18. Method for the determination of an analyte in a sample, characterised in that the sample is brought into contact with a reagent which contains a compound of the structural formulae (I) as defined in Claim 1 and/or (II) as defined in Claim 1, and the analyte is determined by means of a change of the detectable properties of the reagent.

19. Reagent for the determination of an analyte in a sample, characterised in that it contains at least one compound of the structural formulae (I) as defined in Claims 22 to 29 and/or (II) as defined in Claims 22 to 29.

20. Reagent kit, characterised in that it contains a reagent as claimed in Claim 19 in addition to other test compounds.

21. Reagent kit as claimed in Claim 20, characterised in that it contains accompanying substances which modify the molecular properties of compounds of the structural formula (I) and/or (II).

22. Compounds of the general structural formulae (I) or (II): 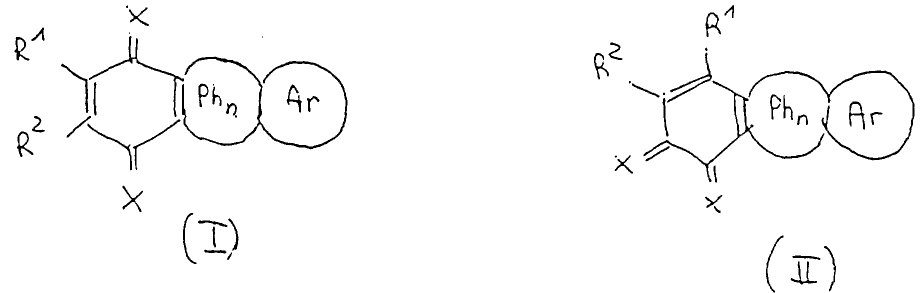 in which R¹, R², X, Ph, n and Ar are as defined in Claim 1 and wherein the compounds contain at least one hydrophilic group selected from (a) sulphonic acid and phosphonic acid groups, as well as salts derived therefrom, (b) sulphuric acid monoester, phosphoric acid monoester and phosphoric acid diester groups, as well as groups derived therefrom, (c) primary, secondary and tertiary amino groups, quaternary ammonium groups, (d) polyhydroxy groups and (e) C²-C³ polyalkyleneoxy and C²-C³ polyalkylenethio groups, except indranthrene monosulphonic acid, indanthrene disulphonic acid, as well as sodium and potassium salts thereof.

23. Compounds as claimed in Claim 22 having the general structural formulae (VIIa) or (VIIb): 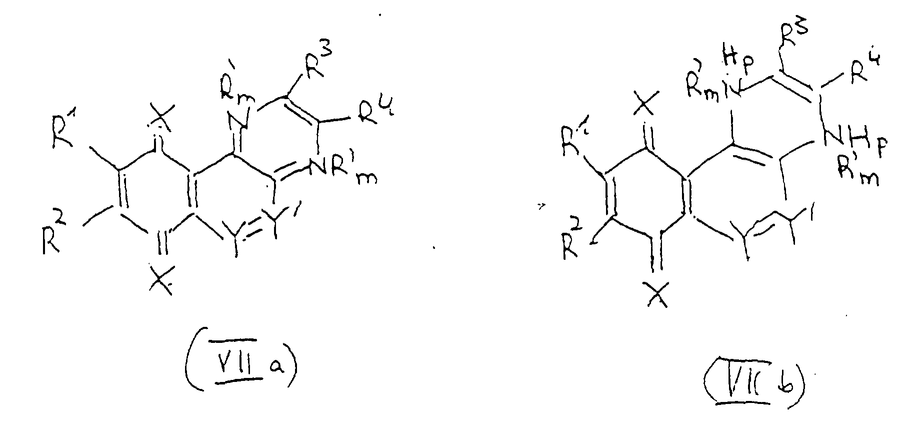 in which R¹, R², X, R', m, p, R³, R⁴, Y and Y' are defined as in Claim 1.

24. Compounds as claimed in Claim 23 having the general structural formulae (VIIIa) or (VIIIb): 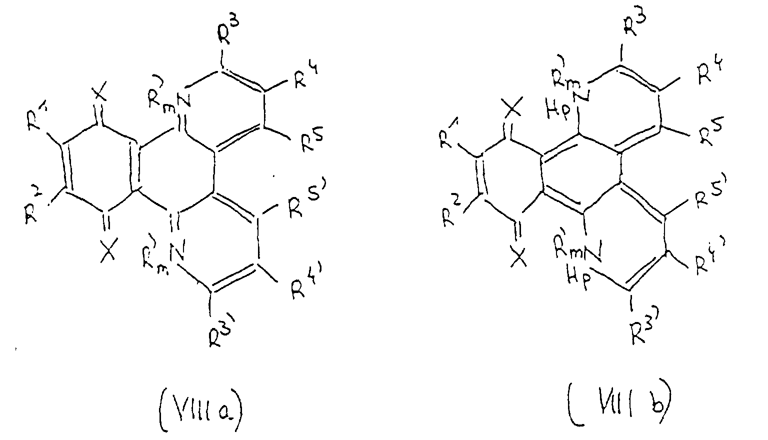 in which R¹, R², X, R', m, p, R³, R⁴, R⁵, R³' R⁴' and R⁵' are defined as in Claim 1.

25. Compounds as claimed in one of the Claims 22 to 24, characterised in that X is the same as 0.

26. Compounds as claimed in one of the Claims 22 to 25, characterised in that at least one of the residues R¹ and R² comprises an aromatic or heteroaromatic ring system.

27. Compounds as claimed in one of the Claims 23 and 25 to 26, characterised in that at least one of the residues R³ and R⁴ comprises an aromatic or heteroaromatic ring system.

28. Compounds as claimed in one of the Claims 23 and 25 to 27, characterised in that Y and Y' each denote CR⁶ and R⁶ has the meaning stated in Claim 1.

29. Compounds as claimed in one of the Claims 24 to 28, characterised in that at least one of the residues R³, R⁴, R⁵, R³', R⁴, R⁵' comprises an aromatic or heteroaromatic ring system.

## Revendications

1. Utilisation de composés des formules structurales I et/ou II ou de sels de ceux-ci pour la préparation de réactifs de mise en évidence pour un procédé pour déterminer un analyte : 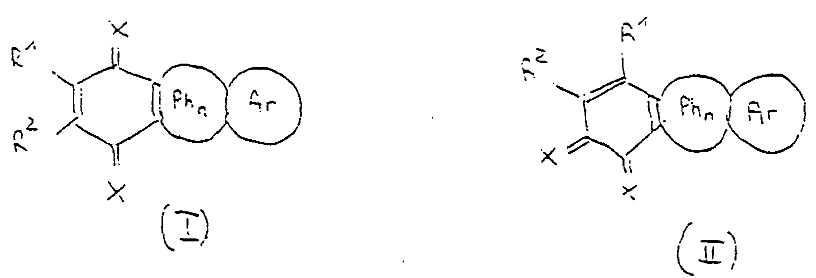 où R¹ et R² sont dans chaque cas indépendamment l'un de l'autre l'hydrogène, des halogènes ou des restes organiques,
X représente O, S, C(Acc)₂, CH(Acc) ou N(Acc) et Acc est un groupe attracteur d'électrons,
Ph représente un cycle phényle éventuellement substitué,
n est un nombre entier de 0 à 4 et
Ar représente un groupe des formules structurales IIIa, IIIb, IVa, IVb, Va, Vb, VIa ou VIb : 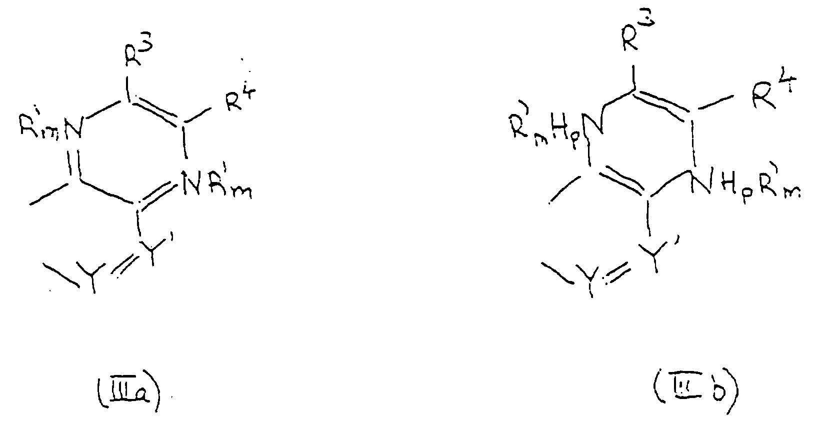 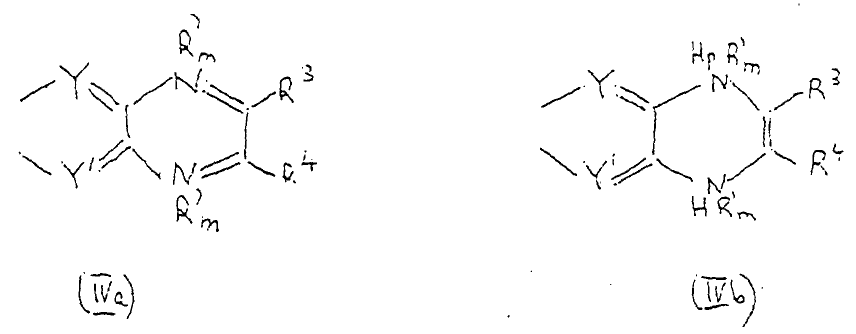 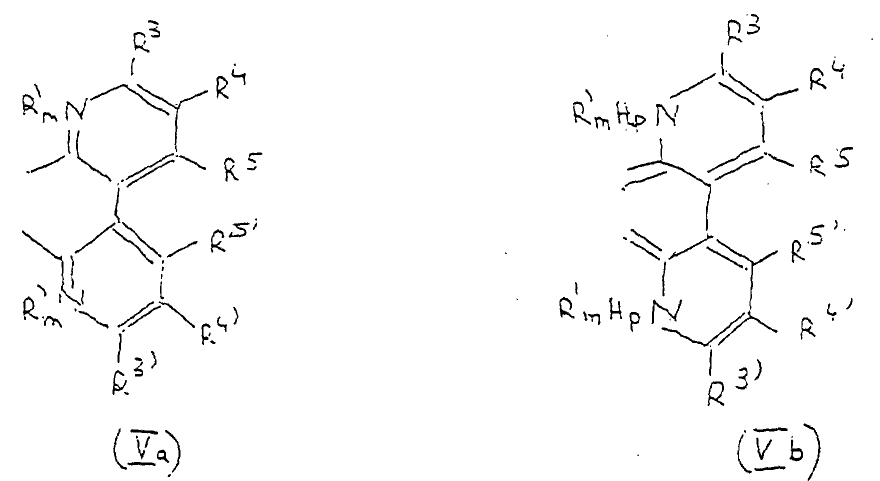 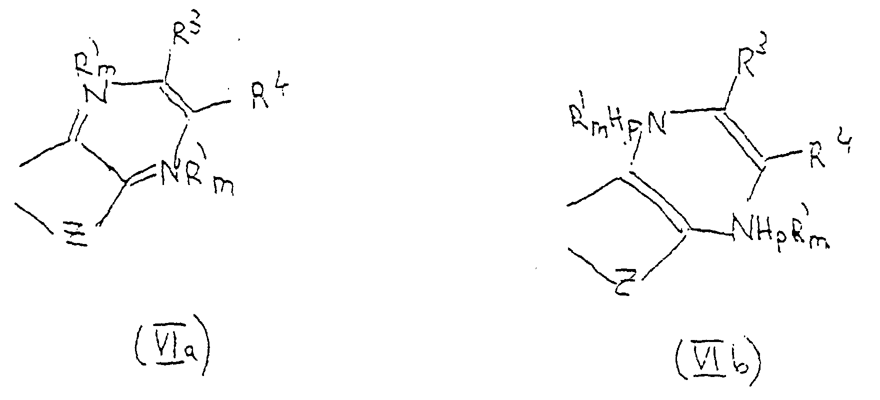 où R³, R³', R⁴, R⁴', R⁵ et R⁵' sont dans chaque cas indépendamment les uns des autres l'hydrogène, un halogène ou des restes organiques, Y et Y' représentent dans chaque cas indépendamment l'un de l'autre N ou CR⁶, où R⁶ est l'hydrogène, un halogène ou un reste organique,
Z est NR, O ou S, où R est un reste organique ou l'hydrogène,
R' est dans chaque cas indépendamment un reste alkyle ou aryle éventuellement substitué et
m est dans chaque cas indépendamment 0 ou 1, où, quand m est égal à 1, dans les formules structurales (IIIa), (IVa), (Va) et (VIa) un azote lié à R' porte une charge positive et un contre-ion correspondant est présent, et dans les formules structurales (II-Ib), (IVb), (Vb) et (VIb) p est égal à 0 et où, quand m est égal à 0, p est égal à 1.

2. Utilisation selon la revendication 1 caractérisée en ce que la détermination de l'analyte comprend une réaction rédox où il se produit une modification des propriétés moléculaires des réactifs de mise en évidence qui peut être mise en évidence.

3. Utilisation selon l'une des revendications précédentes pour la détermination d'une activité d'oxydoréductase.

4. Utilisation selon la revendication 3 caractérisée en ce que la détermination a lieu en présence d'un cosubstrat d'oxydoréductase, en particulier PQQ, NAD(P)⁺ ou NAD(P)H/H⁺.

5. Utilisation selon l'une des revendications 3 à 4 caractérisée en ce que l'oxydoréductase est l'analyte à déterminer.

6. Utilisation selon l'une des revendications 3 à 4 caractérisée en ce qu'un substrat d'oxydoréductase est l'analyte à déterminer.

7. Utilisation selon l'une des revendications 1 à 2 pour la détermination d'une activité d'hydrolase.

8. Utilisation selon la revendication 7 caractérisée en ce que la mise en évidence a lieu par le biais d'un système de couplage oxydatif.

9. Utilisation selon l'une des revendications 7 à 8 caractérisée en ce que l'hydrolase est l'analyte à déterminer.

10. Utilisation selon l'une des revendications 7 à 8 caractérisée en ce qu'un substrat d'hydrolase est l'analyte à déterminer.

11. Utilisation selon l'une des revendications précédentes caractérisée en ce que, dans les composés des formules structurales I et II, au moins l'un des restes R¹ et R² comprend un système cyclique aromatique ou hétéroaromatique qui peut éventuellement être substitué.

12. Utilisation selon la revendication 11 caractérisée en ce que R¹ et R² forment ensemble un système cyclique aromatique ou hétéroaromatique.

13. Utilisation selon l'une des revendications précédentes caractérisée en ce que, dans les groupes des formules structurales IIIa, IIIb, IVa, IVb, Va, Vb, VIa et VIb au moins l'un des restes R³, R⁴ et, dans la mesure où ils sont présents, R³', R⁴', R⁵ et R⁵' comprend un système cyclique aromatique ou hétéroaromatique qui peut éventuellement être substitué.

14. Utilisation selon la revendication 13 caractérisée en ce que, dans les groupes des formules structurales IIIa, IIIb, IVa, IVb, VIa et VIb, R³ et R⁴ sont liés entre eux par pontage et de préférence forment ensemble un système cyclique qui présente au moins en partie une structure aromatique ou hétéroaromatique.

15. Utilisation selon la revendication 13 caractérisée en ce que, dans les groupes des formules structurales Va et Vb (a) R³ et R⁴ et/ou R³' et R⁴' ou (b) R⁴ et R⁵ et/ou R⁴' et R⁵' sont dans chaque cas liés entre eux par pontage et de préférence forment ensemble un système cyclique aromatique ou hétéroaromatique qui présente au moins en partie une structure aromatique ou hétéroaromatique.

16. Utilisation selon la revendication 14 ou 15 caractérisée en ce que les restes forment ensemble dans chaque cas un système cyclique naphtalène ou anthraquinone éventuellement substitué.

17. Utilisation selon l'une des revendications précédentes caractérisée en ce que l'on utilise les composés des formules structurales (I) et/ou (II) sous forme de complexes métalliques.

18. Procédé pour déterminer un analyte dans un échantillon, caractérisé en ce que l'on met l'échantillon en contact avec un réactif qui contient un composé des formules structurales (I) définie comme dans la revendication 1 et/ou (II) définie comme dans la revendication 1, et on réalise la détermination de l'analyte par le biais d'une modification de propriétés du réactif qui peuvent être mises en évidence.

19. Réactif pour la détermination d'un analyte dans un échantillon, caractérisé en ce qu'il contient au moins un composé des formules structurales (I) définie comme dans les revendications 22-29 et/ou (II) définie comme dans les revendications 22-29.

20. Kit de réactifs caractérisé en ce qu'il contient un réactif selon la revendication 19 outre d'autres composants de test.

21. Kit de réactifs selon la revendication 20 caractérisé en ce qu'il contient des substances d'accompagnement qui provoquent une modification des propriétés moléculaires de composés de formule structurale (I) et/ou (II).

22. Composés des formules structurales générales (I) ou (II) : 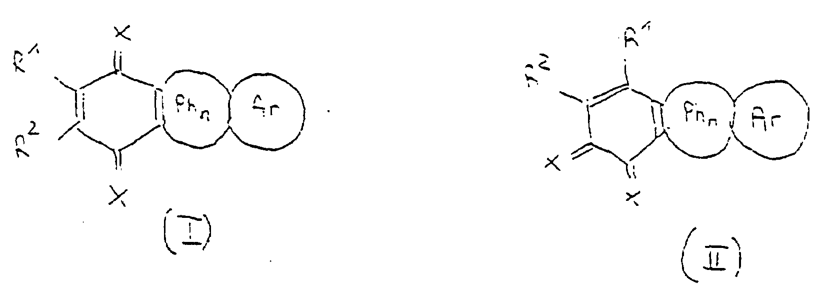 où R¹, R², X, Ph, n et Ar sont définis comme dans la revendication 1 et où les composés contiennent au moins un groupe hydrophile, choisi parmi (a) les groupes acide sulfonique et acide phosphonique ainsi que les sels qui en dérivent, (b) les groupes monoesters d'acide sulfurique, monoesters d'acide phosphorique et diesters d'acide phosphorique ainsi que les sels qui en dérivent, (c) les groupes amines primaires, secondaires et tertiaires, les groupes ammonium quaternaire, (d) les groupes polyhydroxy et (e) les groupes polyalkylèneoxy en C₂-C₃ et polyalkylènethio en C₂-C₃, à l'exception de l'acide indanthrènemonosulfonique, de l'acide indanthrènedisulfonique et de leurs sels de sodium et de potassium.

23. Composés selon la revendication 22 ayant les formules structurales générales (VIIa) ou (VIIb) : 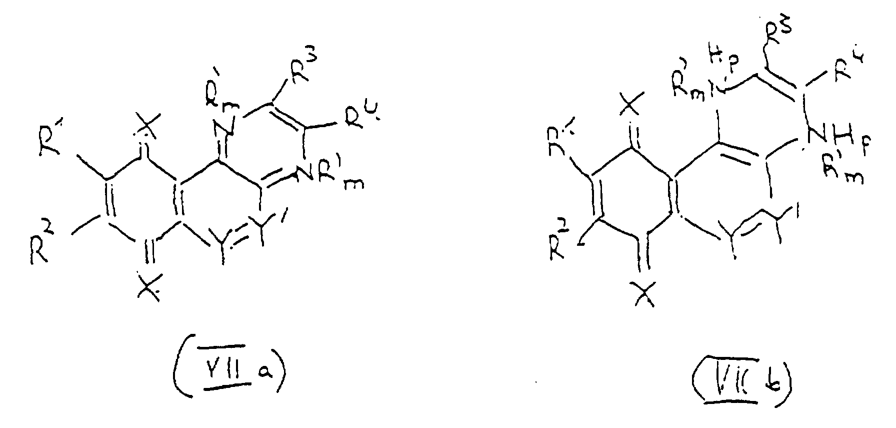 où R¹, R², X, R', m, p, R³, R⁴, Y et Y' sont définis comme dans la revendication 1.

24. Composés selon la revendication 23 ayant les formules structurales générales (VIIIa) ou (VIIIb) : 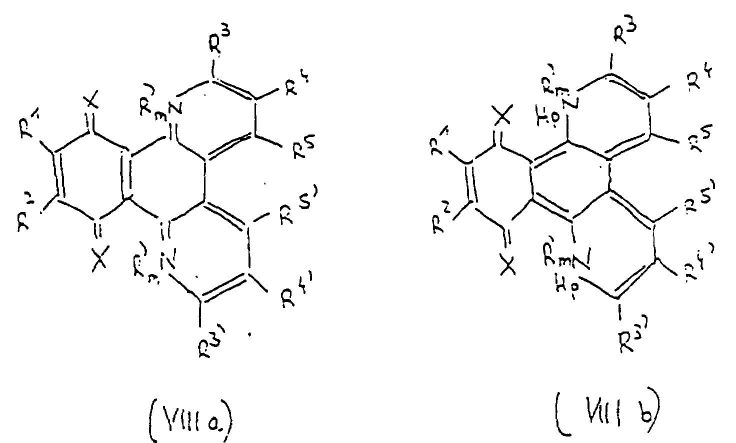 où R¹, R², X, R', m, p, R³, R⁴, R⁵, R³', R⁴' et R⁵' sont définis comme dans la revendication 1.

25. Composés selon l'une des revendications 22 - 24 caractérisés en ce que X représente O.

26. Composés selon l'une des revendications 22 - 25 caractérisés en ce qu'au moins l'un des restes R¹ et R² comprend un système cyclique aromatique ou hétéroaromatique.

27. Composés selon l'une des revendications 23 et 25 - 26 caractérisés en ce qu'au moins l'un des restes R³ et R⁴ comprend un système cyclique aromatique ou hétéroaromatique.

28. Composés selon l'une des revendications 23 et 25 - 27 caractérisés en ce que Y et Y' représentent dans chaque cas CR⁶, où R⁶ possède la signification indiquée dans la revendication 1.

29. Composés selon l'une des revendications 24 - 28 caractérisés en ce qu'au moins l'un des restes R³, R⁴, R⁵, R³', R⁴', R⁵' comprend un système cyclique aromatique ou hétéroaromatique.
